# EUROPEAN PATENT APPLICATION

(11) **EP 4 582 183 A1**
(43) Date of publication of application: **09.07.2025**
(21) Application number: 23860344.3
(22) Date of filing: 29.08.2023
(51) Int. Cl.: B01J 31/28, C07B 61/00, C07C 45/39, C07C 49/08, C07D 207/46, C07D 211/94, C07D 451/00, C07D 451/14

(54) **CATALYST LOADED BODY, CATALYST FOR VAPOR-PHASE OXIDATION OF ORGANIC COMPOUND USING SAME, AND VAPOR-PHASE OXIDATION METHOD**

(30) Priority: 29.08.2022 JP 2022136100
(71) Applicant: NISSAN MOTOR CO., LTD., Kanagawa 221-0023 (JP); Tohoku University, Sendai-shi, Miyagi 980-8577 (JP)
(72) Inventor: ITO, Masashi, Atsugi-shi, Kanagawa 243-0123 (JP); OUME, Ami, Atsugi-shi, Kanagawa 243-0123 (JP); UCHIMURA, Masanobu, Atsugi-shi, Kanagawa 243-0123 (JP); NISHIHARA, Hirotomo, Sendai-shi, Miyagi 980-8577 (JP); YOSHII, Takeharu, Sendai-shi, Miyagi 980-8577 (JP); WAKABAYASHI, Keigo, Sendai-shi, Miyagi 980-8577 (JP); IWABUCHI, Yoshiharu, Sendai-shi, Miyagi 980-8577 (JP); SASANO, Yusuke, Sendai-shi, Miyagi 980-8577 (JP); HIRASAWA, Noriyasu, Sendai-shi, Miyagi 980-8577 (JP); SEGAWA, Ryosuke, Sendai-shi, Miyagi 980-8577 (JP); KONNO, Tomohiro, Sendai-shi, Miyagi 980-8577 (JP)
(74) Representative: Grünecker Patent- und Rechtsanwälte PartG mbB
(86) International application number: PCT/JP2023/031215
(87) International publication number: WO 2024/048582

(57) **Abstract**

Provided is a means capable of oxidizing an organic compound in a gas phase using a nitroxyl radical as a catalyst.

A catalyst support in which a compound having an oxidation-reduction mechanism below and a cocatalyst containing a transition metal are supported on a support: 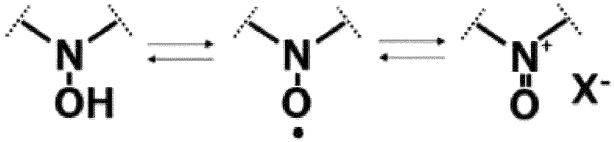 broken line represents a bonding position with another atom.

## Description

### TECHNICAL FIELD

The present invention relates to a catalyst support, and a catalyst for gas phase oxidation and a method for gas phase oxidation of an organic compound using the same.

### BACKGROUND ART

Nitroxyl radicals are stable organic free radical molecules, and are widely used as an oxidation catalyst in the field of organic synthesis. In particular, organic nitroxyl radicals exhibit bidirectional reactivity, that is, oxidation and reduction, and as shown in the following reaction formula, undergo a reaction that generates hydroxylamine by one-electron reduction and a reaction that generates an oxoammonium ion by one-electron oxidation.

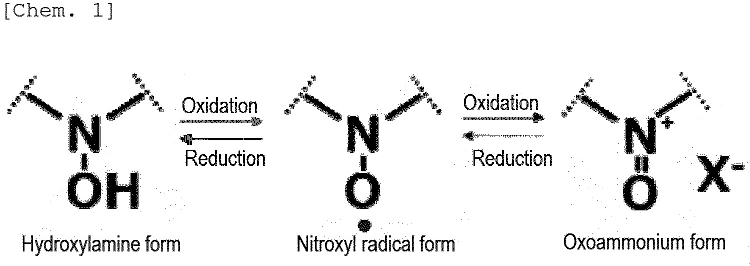

Oxoammonium ions produced by oxidation of nitroxyl radicals have a strong oxidizing power, and give hydroxylamine after two-electron oxidation of a substrate. In an oxidation reaction in which the oxoammonium ions act as active species for oxidation, a catalyst cycle can also be completed by adding a reactant that promotes reoxidation of the hydroxylamine to regenerate the oxoammonium ions.

As such an oxidation catalyst, 2,2,6,6-tetramethylpiperidine-1-oxyl (TEMPO) is conventionally known. Also, 2-azaadamantane-N-oxyl (AZADO) has also been proposed as a catalyst that has improved a major steric hindrance of TEMPO (see WO 2006/001387). AZADO has enabled highly efficient oxidation of a variety of alcohols with significant steric hindrance to the corresponding carbonyl compounds.

On the other hand, in recent years, an oxidation reaction using oxygen in the air as an oxidizing agent has attracted great attention from the viewpoint of reducing the environmental load. For example, Iwabuchi et al. have reported that oxidation of various alcohols can be promoted with high efficiency in a reaction system of AZADO/NaNO₂/O₂ using AZADO or 5-fluoro-AZADO (see M. Shibuya, Y. Osada, Y. Sasano, M. Tomizawa, Y. Iwabuchi, J. Am. Chem. Soc. 2011, 133, 6497-6500).

All the techniques described in WO 2006/001387 and M. Shibuya, Y. Osada, Y. Sasano, M. Tomizawa, Y. Iwabuchi, J. Am. Chem. Soc. 2011, 133, 6497-6500 described above are techniques of oxidizing an alcohol to a corresponding aldehyde or ketone in a liquid phase system, and a technique of oxidizing an alcohol in a gas phase using a nitroxyl radical such as TEMPO or AZADO as a catalyst has not been reported so far.

### SUMMARY OF INVENTION

### Technical Problem

In a case of a reaction in the liquid phase system described in the documents described above, it is necessary to separate a target product and a catalyst from a solvent after completion of a reaction and further purify the target product, which has been a problem in terms of time and cost.

Therefore, an object of the present invention is to provide a technique capable of oxidizing an organic compound in a gas phase using a nitroxyl radical as a catalyst.

### Solution to Problem

The present inventors have conducted intensive studies to solve the above problem. As a result, the present inventors have found that the above problem can be solved by a catalyst support in which a compound having an oxidation-reduction mechanism represented by the above reaction formula is supported on a support together with a cocatalyst, and have completed the present invention.

That is, in one aspect, there is a catalyst support in which a compound having an oxidation-reduction mechanism below and a cocatalyst containing a transition metal are supported on a support:

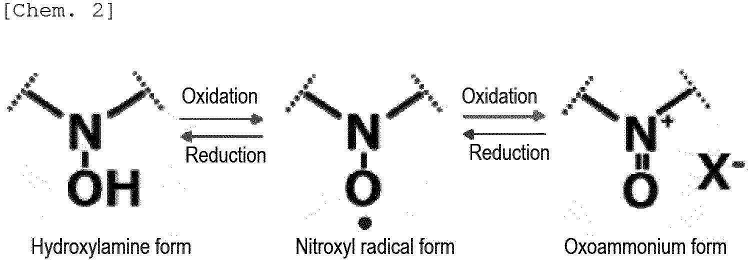

In the formula, X⁻ is a counteranion, and a broken line represents a bonding position with another atom.

### Effect of Invention

According to the present invention, an organic compound can be oxidized in a gas phase using a nitroxyl radical as a catalyst.

### BRIEF DESCRIPTION OF DRAWINGS

FIG. 1 is a cyclic voltammogram obtained by measuring a standard redox potential (E⁰ @25°C) between a nitroxyl radical form and an oxoammonium form by a method using cyclic voltammetry for the four compounds (a) TEMPO, (b) AZADO, (c) 1-methyl AZADO, and (d) 1,3-dimethyl AZADO.
FIG. 2 is an XRD pattern showing a result of XRD measurement of AZADO/GMS prepared in Examples together with measurement results for AZADO and GMS.
FIG. 3 is an ESR spectrum showing results of ESR measurement of AZADO/GMS prepared in Examples.
FIG. 4 is a graph showing a result obtained by performing a gas phase oxidation reaction of 2-propanol in a batch system at 80°C using AZADO/GMS prepared in Examples as a catalyst (the amount of acetone produced over 24 hours after the start of a reaction) together with the results of a case of using only GMS as a catalyst and a case of not using a catalyst (Blank).
FIG. 5 is an XRD pattern showing results of XRD measurement of AZADO-Cu(NO₃)₂/GMS, AZADO-CuI/GMS, and AZADO-Fe(NO₃)₃/GMS prepared in Examples.
FIG. 6 is an XRD pattern showing results of XRD measurement of AZADO-Cu(NO₃)₂/GMS prepared in Examples, together with the measurement results for Cu (NO₃)₂·3H₂O and Cu₂(NO₃)(OH)₃.
FIG. 7 is a graph showing results obtained by performing a gas phase oxidation reaction of 2-propanol in a batch system using AZADO-Cu(NO₃)₂/GMS, Cu(NO₃)₂/GMS, and AZADO/GMS prepared in Examples as catalysts (the amount of acetone produced at 80°C for 24 hours after the start of a reaction) together with a result in a case of using only GMS as a catalyst.
FIG. 8 is a graph showing a result obtained by applying AZADO-Cu(NO₃)₂/GMS and AZADO-CuI/GMS prepared in Examples used as catalysts to a gas phase oxidation reaction of 2-propanol at normal temperature (30°C) in a batch system with temporal change of TON (Turnover Number) as an index of catalytic activity.
FIG. 9 is a graph showing a result obtained by performing a gas phase oxidation reaction of 2-propanol (O₂ including 1.0 mmol/L of 2-propanol was circulated at 20 mL/min) in a flow system at room temperature (24°C) using AZADO-Cu(NO₃)₂/GMS prepared in Examples as a catalyst as a temporal change of TOF (Turnover Frequency) as an index of catalytic activity.
FIG. 10 is a graph showing a result obtained by performing a gas phase oxidation reaction of 2-propanol (30°C) in a batch system using AZADO-Cu(bpy)/GMS prepared in Examples as a catalyst (temporal change of TON) together with results obtained in cases of using AZADO-Cu(NO₃)₂/GMS, AZADO-CuI/GMS, or AZADO/GMS as a catalyst.
FIG. 11 is a graph showing a result obtained by performing a gas phase oxidation reaction of 2-propanol (O₂ including 1.8 mmol/L of 2-propanol was circulated at 50 mL/min) in a flow system at 30°C using AZADO-Cu(bpy)/GMS prepared in Examples as a catalyst as a temporal change of TOF.
FIG. 12 is a graph comparing TON's at 24 hours after the start of a reaction, when performing a gas phase oxidation reaction of 2-propanol in a batch system at 30°C using AZADO-Cu(bpy)/GMS and TEMPO-Cu(bpy)/GMS prepared in Examples as catalysts.
FIG. 13 is a graph comparing TON's at 24 hours after the start of a reaction, when performing a gas phase oxidation reaction of ethanol in a batch system at 30°C using AZADO-Cu(bpy)/GMS and TEMPO-Cu(bpy)/GMS prepared in Examples as catalysts.
FIG. 14 is a graph comparing the TON at 8 hours after the start of a reaction, when introducing N₂ including 1.8 mmol/L of 2-propanol into a reaction vessel and performing a reaction at 30°C for 8 hours in a batch system using AZADO-Cu(bpy)/GMS as a catalyst, with that of a case under an O₂ atmosphere.
FIG. 15 is a graph comparing a temporal change of TON when introducing a synthetic air including 1.8 mmol/L of 2-propanol into a reaction vessel and performing a reaction at 30°C for 24 hours in a batch system using AZADO-Cu(bpy)/GMS as a catalyst, with that of a case under an O₂ atmosphere.
FIG. 16 is a graph showing temporal changes of TON obtained for a catalyst using various carbon materials as supports when performing a gas phase oxidation reaction of 2-propanol in a batch system using a catalyst in which AZADO and Cu(bpy) are supported on various carbon materials and oxides.
FIG. 17 is a graph showing temporal changes of TON obtained for a catalyst using various oxides as supports when performing a gas phase oxidation reaction of 2-propanol in a batch system using a catalyst in which AZADO and Cu(bpy) are supported on various carbon materials and oxides.
FIG. 18 is a scatter diagram obtained by plotting TON against each of a specific surface area, a total pore volume, the number of edge sites, and a spin density of each carbon material at 24 hours after the start of a reaction, obtained using catalysts using various carbon materials as supports.
FIG. 19 is a scatter diagram obtained by arranging TON's at 24 hours after the start of a reaction obtained by using catalysts using various oxides as supports according to a specific surface area of each oxide, and plotting the TON's against a specific surface area of each oxide support at 24 hours after the start of a reaction for the purpose of comparison with a case of using a carbon support. FIG. 19 also shows plots in cases of using various carbon supports.

### DESCRIPTION OF EMBODIMENTS

Hereinafter, modes for carrying out the present invention will be described.

### <<Catalyst Support>>

One aspect of the present invention is a catalyst support in which a compound having an oxidation-reduction mechanism (hereinafter, also referred to as a "compound of the present aspect") below and a cocatalyst containing a transition metal (hereinafter, also referred to as a "cocatalyst of the present aspect") are supported on a support (hereinafter, also referred to as a "support of the present aspect").

### <Compound of the Present Aspect >

The compound of the present aspect is sufficient to have the above-described redox mechanism, and the specific structure thereof is not limited at all. In addition, the compound of the present aspect may be a hydroxyamine form, a nitroxyl radical form, or an oxoammonium form in the redox mechanism described above. Among them, a hydroxyamine form or a nitroxyl radical form is preferable, and a nitroxyl radical form is particularly preferable.

In addition, from the viewpoint of exhibiting excellent catalytic activity when used as an oxidation catalyst explained later, the standard redox potential (25°C) between the nitroxyl radical form and the oxoammonium form for the compound of the present aspect is preferably +100 mV to +1000 mV [Ag/Ag⁺], and more preferably +130 mV to +600 mV [Ag/Ag⁺]. The standard redox potential is measured by cyclic voltammetry (CV) under a temperature condition of 25°C. At this time, the measurement is performed by a three-electrode method using a glassy carbon electrode (inner diameter: 3 mm) as a working electrode, a reference electrode (Ag/Ag⁺), and a counter electrode (platinum wire), and the sweep rate of the potential is 50 mV/s. It is noted that the hydroxyamine forms of the compounds of the present aspect are generally unstable and rapidly oxidized into nitroxyl radical forms. Thus, the value of the standard redox potential measured by the above method is the value between the nitroxyl radical form and the oxoammonium form.

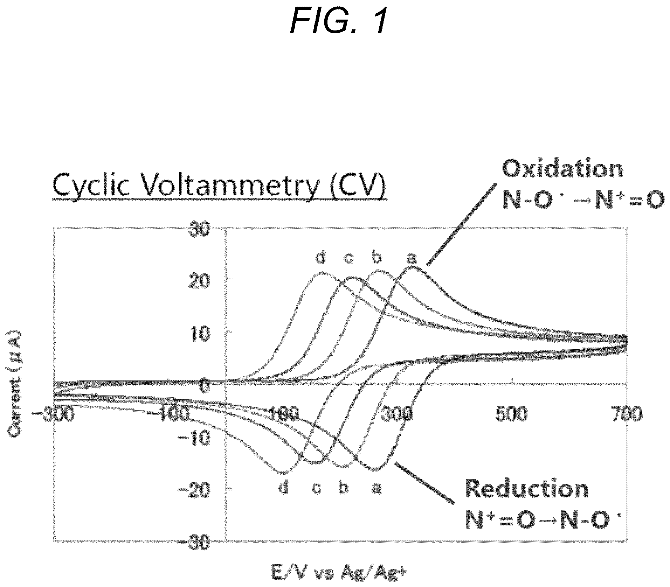

Here, Fig. 1 shows a cyclic voltammogram obtained by measuring the standard redox potential (E°@25°C) between the nitroxyl radical form and the oxoammonium form by the method using cyclic voltammetry described above for four compounds of (a) TEMPO, (b) AZADO, (c) 1-methyl AZADO, and (d) 1,3-dimethyl AZADO. As shown in Fig. 1, the E⁰ values for these compounds were (d) 136 mV < (c) 186 mV < (b) 236 mV < (a) 294 mV, respectively. In the cyclic voltammogram shown in Fig. 1, the cyclic voltammogram was stably maintained even after repeating 100 or more cycles of measurement, and thus it is observed that the compound of the present aspect has very high durability as an oxidizing agent.

From the viewpoint of catalytic activity, the nitroxyl radical form of the compound of the present aspect is preferably represented by the following Chemical Formula 1.

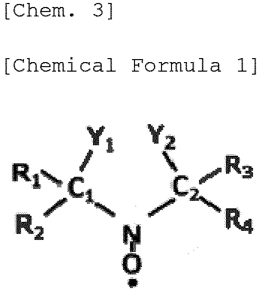

In Chemical Formula 1, R₁ to R₄, Y₁, and Y₂ are each independently a hydrogen atom or an optionally substituted monovalent organic group. Here, the monovalent organic group is not particularly limited, and examples thereof include a substituted or unsubstituted alkyl group, a substituted or unsubstituted alkenyl group, a substituted or unsubstituted alkynyl group, a substituted or unsubstituted aryl group, a substituted or unsubstituted heteroaryl group, and the like. R₁ to R₄, Y₁, and Y₂ may be the same or different.

Each of the alkyl group, the alkenyl group, and the alkynyl group as the monovalent organic group may be linear, branched, or cyclic, but is preferably linear. The number of carbon atoms in the alkyl group is not particularly limited, but is preferably 1 or more and 20 or less, more preferably 1 or more and 10 or less, and still more preferably 1 or more and 4 or less. The number of carbon atoms in each of the alkenyl group and the alkynyl group is not particularly limited, but is preferably 2 or more and 20 or less, more preferably 2 or more and 10 or less, and still more preferably 2 or more and 4 or less.

The aryl group as the monovalent organic group is a group derived from a hydrocarbon ring having aromaticity as a part or the whole. When the aryl group includes two or more hydrocarbon rings having aromaticity as a part or a whole, these rings may be bonded or fused to each other by a single bond. When the aryl group contains two or more hydrocarbon rings having aromaticity as a part or a whole, one atom may also serve as a ring-forming atom of any two of these rings to form a spiro ring. The number of carbon atoms in the aryl group is not particularly limited, but is preferably 6 or more and 30 or less. The number of carbon atoms in the aryl group is more preferably 6 or more and 12 or less, and still more preferably 6. In particular, the aryl group is preferably a monovalent group derived from an aromatic hydrocarbon ring having 6 or more ring-forming atoms.

The heteroaryl group as a monovalent organic group is a group derived from a heterocyclic ring having aromaticity as a part or as a whole. When the heteroaryl group includes two or more heterocyclic rings having aromaticity as a part or a whole, some or all of these rings may be bonded to each other by a single bond. When the heteroaryl group includes two or more heterocyclic rings having aromaticity as a part or a whole, or other rings, these rings may be fused to each other. When the heteroaryl group includes two or more heterocyclic rings having aromaticity as a part or the whole, or other rings, one atom may also serve as a ring-forming atom of these rings. The heteroatom included in the heteroaryl group is not particularly limited, and examples thereof include one or more heteroatoms (for example, a nitrogen atom (N), an oxygen atom (O), a phosphorus atom (P), a sulfur atom (S), a silicon atom (Si), a selenium atom (Se), and a germanium atom (Ge)) and the like as a ring-forming atom. The number of carbon atoms in the heteroaryl group is preferably 3 or more and 30 or less. In addition, the number of ring-forming atoms of the heteroaryl group is not particularly limited, but is preferably 5 or more and 30 or less. Furthermore, the number of ring-forming atoms of the heteroaryl group is more preferably 5 or more and 14 or less, and still more preferably 5 or more and 13 or less. The number of heteroatoms in the heteroaryl group is not particularly limited, but is preferably 1 or more and 3 or less. In addition, the number of heteroatoms of the heteroaryl group is more preferably 1 or more and 2 or less, and still more preferably 1. In particular, the heteroaryl group is preferably a monovalent group derived from an aromatic heterocyclic ring having 5 or more ring-forming atoms.

Each of the alkyl group, the alkenyl group, the alkynyl group, the aryl group, and the heteroaryl group as the monovalent organic group may be a substituted group. The substituent is not particularly limited, and examples thereof include an alkyl group, an alkoxy group, an alkenyl group, an alkynyl group, a halogen atom, a hydroxy group, a carboxy group, a cyano group, an amino group, a carbamoyl group, and the like. Among them, the substituent is preferably an alkyl group or a halogen atom, and more preferably a methyl group, an ethyl group, or a fluorine atom. The substituent is more preferably a methyl group or a fluorine atom. The substituent does not substitute the same kind of group. For example, a substituent that substitutes an alkyl group does not include an alkyl group.

In addition, in Chemical Formula 1, from the viewpoint of excellent catalytic activity, preferably, Y₁ and Y₂ may be bonded to each other to form an optionally substituted nitrogen-containing monocyclic ring containing a 5- or 6-membered ring, and further, R₁ and R₃ may be bonded to each other to form a nitrogen-containing bicyclic ring containing a 5- or 6-membered ring which may be substituted, or two rings constituting the nitrogen-containing bicyclic ring may further be crosslinked to form a nitrogen-containing tricyclic ring containing a 5- or 6-membered ring which may be substituted. That is, in a preferred embodiment of the present aspect, the nitroxyl radical form of the compound of the present aspect is represented by the following Chemical Formula 2.

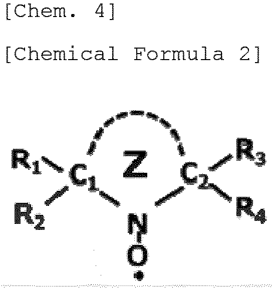

In Chemical Formula 2, R₁ to R₄ are each independently a hydrogen atom or an optionally substituted monovalent organic group, Z is an optionally substituted nitrogen-containing monocyclic ring containing a 5- or 6-membered ring, and in this case, R₁ and R₃ may be bonded to each other to form an optionally substituted nitrogen-containing bicyclic ring containing a 5- or 6-membered ring, or two rings constituting the nitrogen-containing bicyclic ring may be further crosslinked to form an optionally substituted nitrogen-containing tricyclic ring containing a 5- or 6-membered ring.

Examples of the nitrogen-containing monocyclic ring as Z include aliphatic heterocyclic rings such as a pyrrolidine ring, a piperidine ring, a morpholine ring, and a pyrroline ring, and aromatic heterocyclic rings such as a pyrrole ring and an imidazole ring. In addition, examples of the nitrogen-containing bicyclic ring formed by further bonding R₁ and R₃ to each other include a 9-azabicyclo[3.3.1]nonane ring and a 9-aza-3-oxabicyclo[3.3.1]nonane ring. Further, examples of the nitrogen-containing tricyclic ring formed by further crosslinking the two rings constituting the nitrogen-containing bicyclic ring include a 2-azaadamantane ring, a 2,6-diazaadamantane ring, a 6-aza-2-oxaadamantane ring, and a 9-aza-6-noradamantane ring. Among them, from the viewpoint of excellent catalytic activity, the compound of the present aspect preferably has a nitrogen-containing monocyclic ring, a nitrogen-containing bicyclic ring, or a nitrogen-containing tricyclic ring, more preferably has a nitrogen-containing monocyclic ring or a nitrogen-containing tricyclic ring, and particularly preferably has a nitrogen-containing tricyclic ring. In addition, the compound of the present aspect preferably has an aliphatic heterocyclic ring which is a nitrogen-containing monocyclic ring, a 2-azaadamantane ring which is a nitrogen-containing tricyclic ring, or a 9-aza-6-noradamantane ring, and more preferably has a piperidine ring which is a nitrogen-containing monocyclic ring or a 2-azaadamantane ring which is a nitrogen-containing tricyclic ring.

The nitrogen-containing monocyclic ring formed in Chemical Formula 2 or the nitrogen-containing bicyclic ring or the nitrogen-containing tricyclic ring that can be formed in Chemical Formula 2 is an optionally substituted ring structure. The substituent capable of substituting these ring structures is not particularly limited, and examples thereof include an alkyl group, an alkoxy group, an alkenyl group, an alkynyl group, a halogen atom, a hydroxy group, a carboxy group, a carbonyl group, a cyano group, an amino group, a carbamoyl group, a tosyl group, a (halogenated) acylamino group, a phosphate group, and the like. Among them, the substituent is more preferably a methyl group, an ethyl group, a methoxy group, an ethoxy group, a fluorine atom, a hydroxy group, a carboxy group, or a tosyl group.

Here, when the compound of the present aspect does not contain the ring structure or contains a nitrogen-containing monocyclic ring, R₁ to R₄ are each independently preferably a hydrogen atom or an optionally substituted alkyl group, more preferably a hydrogen atom or an unsubstituted alkyl group, further preferably a hydrogen atom or an alkyl group having 1 to 4 carbon atoms, and particularly preferably a hydrogen atom, a methyl group, or an ethyl group. From the viewpoint of catalytic activity, when the compound of the present aspect contains the nitrogen-containing bicyclic ring or the nitrogen-containing tricyclic ring, R₂ and R₄ are each independently preferably a hydrogen atom or an optionally substituted alkyl group, more preferably a hydrogen atom or an unsubstituted alkyl group, still more preferably a hydrogen atom or an alkyl group having 1 to 4 carbon atoms, and particularly preferably a hydrogen atom, a methyl group, or an ethyl group.

An example of the compound of the present aspect is as follows (indicated by nitroxyl radical form). For some compounds, the standard redox potential (25°C) values between the nitroxyl radical form and the oxoammonium form described above are also shown. In view of the mechanism by which the catalyst support according to the present aspect exhibits excellent catalytic activity, it can be said that all of the compounds shown below exhibit high catalytic activity.

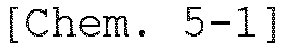

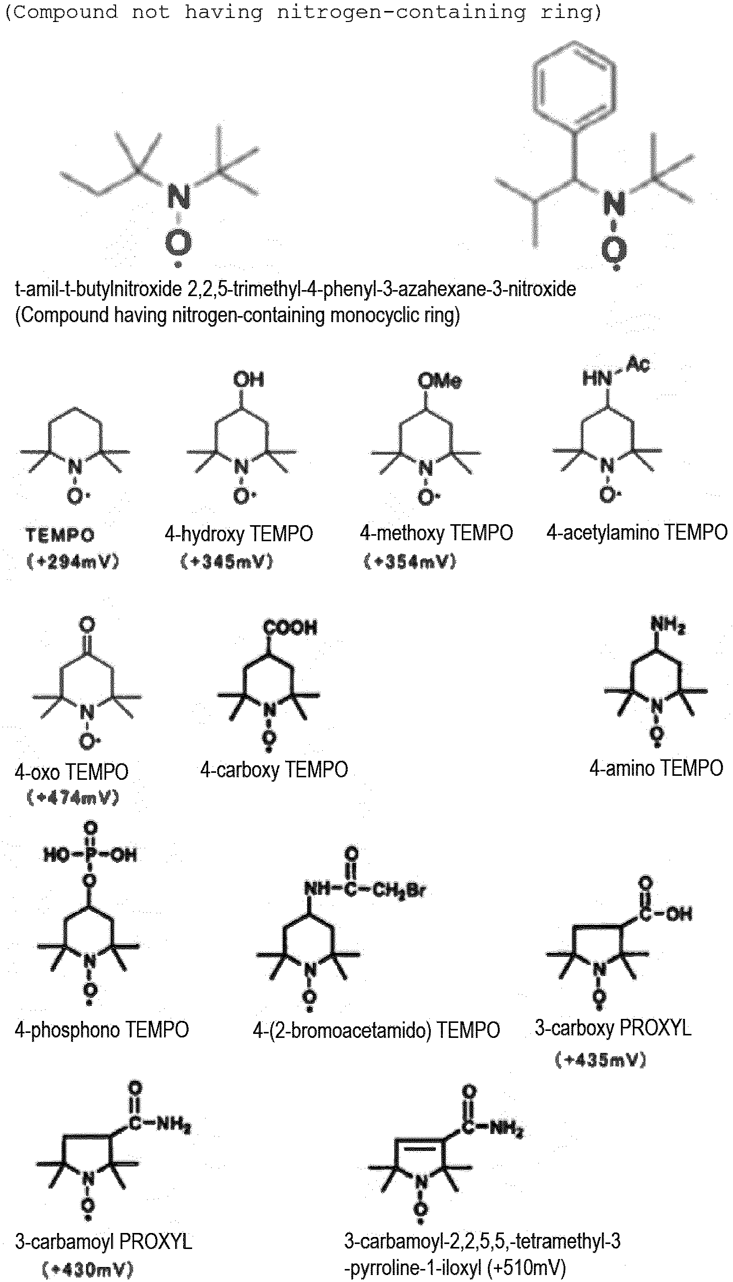

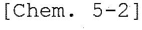

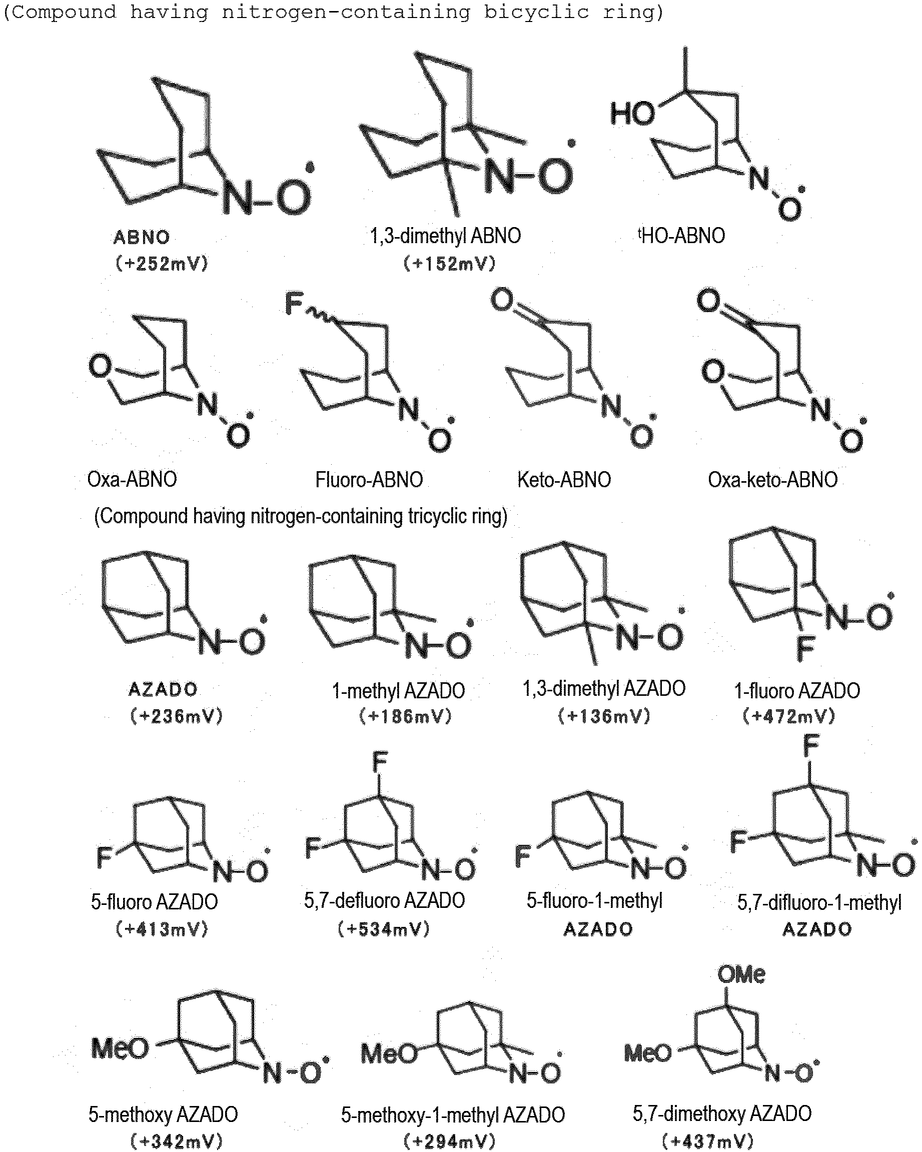

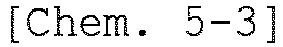

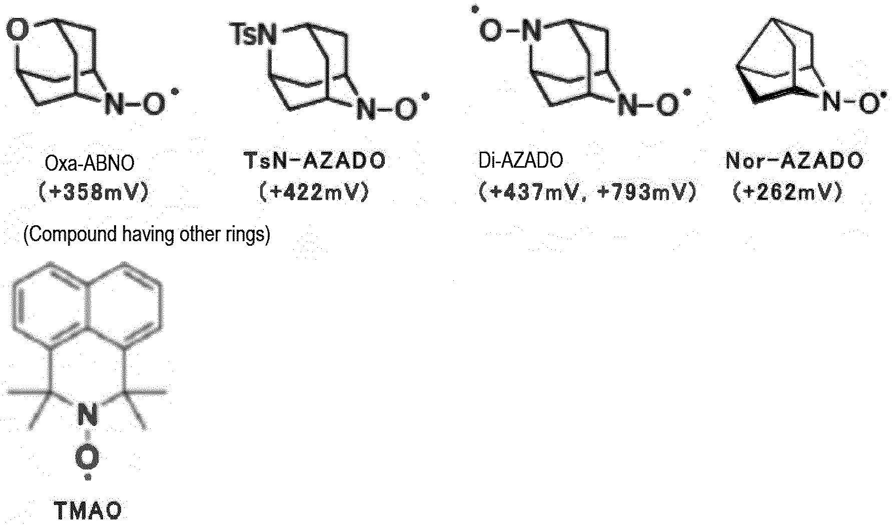

In view of the value of E⁰ described above, when heteroatoms are introduced into a structure of TEMPO, ABNO, AZADO, or the like constituting the skeleton of the compound, the redox characteristics of the compound of the present aspect shift to the high potential side. From this, it is considered that the oxidizing power as the potential of the corresponding oxoammonium form increases.

Among the above compounds, from the viewpoint of catalytic activity, the compounds are preferably TEMPO, 4-hydroxy TEMPO, 4-methoxy TEMPO, 4-acetylamino TEMPO, 4-oxoTEMPO, 4-carboxy TEMPO, 4-amino TEMPO, 4-phosphono TEMPO, 4-(2-bromoacetamido) TEMPO, AZADO, 1-methyl AZADO, 1,3-dimethyl AZADO, 1-fluoro AZADO, 5-fluoro AZADO, 5,7-difluoro AZADO, 5-fluoro-1-methyl AZADO, 5,7-difluoro-1-methyl AZADO, 5-methoxy AZADO, 5-methoxy-1-methyl AZADO, 5,7-dimethoxy AZADO, oxa-AZADO, TsN-AZADO, and di-AZADO or Nor-AZADO, more preferably, AZADO, 1-methyl AZADO, 1,3-dimethyl AZADO, 1-fluoro AZADO, 5-fluoro AZADO, 5,7-difluoro AZADO, 5-fluoro-1-methyl AZADO, 5,7-difluoro-1-methyl AZADO, 5-methoxy AZADO, 5-methoxy-1-methyl AZADO, 5,7-dimethoxy AZADO, oxa-AZADO, TsN-AZADO, di-AZADO, Nor-AZADO, TEMPO or 4-acetylamino TEMPO, further preferably AZADO, 1-methyl AZADO, 1,3-dimethyl AZADO, 1-fluoro AZADO, 5-fluoro AZADO, 5,7-difluoro AZADO, 5-fluoro-1-methyl AZADO, 5,7-difluoro-1-methyl AZADO, 5-methoxy AZADO, 5-methoxy-1-methyl AZADO, 5,7-dimethoxy AZADO, TEMPO or 4-acetylamino TEMPO, and particularly preferably AZADO (2-azaadamantane-N-oxyl).

### <Cocatalyst of the present aspect>

In the catalyst support of the present aspect, the above-mentioned compound is supported on a support together with a cocatalyst. Here, the cocatalyst of the present aspect contains a transition metal. It is noted that the cocatalyst may be in the form of a simple substance of a transition metal, or in the form of a transition metal compound (for example, a salt or a complex). The transition metal that can be contained in the cocatalyst of the present aspect is not particularly limited as long as the transition metal is a metal element classified into Group 3 elements to Group 12 elements in the periodic table of elements. Among them, from the viewpoint of excellent catalytic activity, the transition metal preferably contains one or more selected from the group consisting of Ag, Au, Pt, Pd, Ni, Mn, Fe, Ti, Al, Zn, and Cu, and more preferably contains ions (salts) thereof. In addition, the transition metal further preferably contains Cu, particularly preferably contains Cu ions (salts), and most preferably contains divalent Cu ions (Cu(II) ions). In the meantime, it has been reported that in the past, in reactions in liquid phase systems using AZADO as a catalyst, higher activity is obtained when Cu(I) ions are used than when Cu(II) ions are used. In contrast, it has been confirmed that in reactions using catalyst supports of the present aspect, a catalyst containing divalent Cu(II) ions show higher activity than those containing monovalent Cu(I) ions.

When the cocatalyst contains an ion (salt) of a transition metal, the counteranion of the ion is not particularly limited. Examples of the salt containing a transition metal as a cocatalyst include a sulfate, a sulfite, a hyposulfite, a persulfate, a thiosulfate, a carbonate, a phosphate, pyrophosphoric acid, a hydrochloride, a nitrate, a nitrite, a halide (for example, fluoride, chloride, bromide, or iodide), a trifluoromethanesulfonate, and the like. It is preferable that the transition metal is contained in the form of divalent ions as mentioned above.

The supported amounts of the compound and the transition metal in the catalyst support of the present aspect are not particularly limited, but from the viewpoint of excellent catalytic activity, the molar ratio of the supported amounts of these components used is preferably 1:2 to 4:1 (compound : transition metal), and more preferably 2:3 to 3:2. Further, although details of the support will be described later, a coverage of a surface area of the support by the compound and the cocatalyst is 10% to 300% from the viewpoint of catalytic activity. A value of the coverage can be determined by approximating the size of the compound according to the present aspect and the cocatalyst (metal and ligand) to a circle or a rectangle, calculating the coverage of a surface of a support by each component, and summing all of them. For example, taking AZADO-Cu(bpy) as an example, AZADO can be approximated to the same size as cyclohexane (a square with sides of 5 Å), and Cu of Cu(bpy) can be approximated to a circle with a radius of 1.28 Å and bpy thereof can be approximated to a rectangle with dimensions of 5 Å × 8.5 Å. Here, in a case in which the value of the coverage exceeds 100%, it is considered that a complete covering layer of the component is formed on the surface of the support, and then more of the component is laminated on the complete covering layer. The value of the coverage can be controlled by adjusting a blending amount of each component when producing the catalyst support.

In the catalyst support of the present aspect, it is essential that the above-described compound and transition metal are supported on a support, but other components may be further supported as necessary. Examples of the other components include a ligand. When the ligand is further supported, the catalytic activity can be further improved. In this case, the transition metal and the ligand can form a complex. As the ligand, a conventionally known ligand can be appropriately used, and a compound containing a nitrogen-containing 6-membered ring is preferable. Among them, the ligand is more preferably a pyridine ring-containing compound, and further preferably a bipyridine compound, a terpyridine compound, or a phenanthroline compound. Specific examples of these compounds include 2,2'-bipyridine(bpy), 2,6-di(2-pyridyl)pyridine, 1,10-phenanthroline, 4,7-biphenyl-1,10-phenanthroline, 1,7-phenanthroline, bathocuproine, bathocuproine sulfonic acid, and the like, and among these, 2,2'-bipyridine(bpy) is particularly preferable. The supported amount in a case of using a ligand is also not particularly limited, and from the viewpoint of excellent catalytic activity, a molar ratio between the cocatalyst and the ligand is preferably 3:1 to 1:3 (cocatalyst:ligand), and more preferably 2:1 to 1:2.

### <Support of the Present Aspect>

A specific configuration of the support of the present aspect is also not particularly limited, and conventionally known knowledge can be appropriately referred to. Examples of the support include a metal oxide or a carbon material, and a carbon material is more preferable. Examples of the metal oxide that can be used as a support include silica (such as silica gel or fumed silica), alumina (such as α alumina, θ alumina, γ alumina, δ alumina, and β alumina), titania, ceria, silica alumina, zirconia, magnesia, zeolite, and the like. In a case in which a metal oxide is used as a support, the metal oxide is preferably alumina, titania, or ceria, and more preferably alumina.

Also, the carbon material used as a support is preferably a porous carbon material. The porous carbon material contains carbon as a main component. Here, the expression "contains carbon as a main component" is a concept including both of being composed only of carbon and being substantially composed of carbon, and elements other than carbon may be contained. The expression "being substantially composed of carbon" means that 80 mass% or more of the whole, preferably 95 mass% or more of the whole, and more preferably 98 mass% or more of the whole (upper limit: 100 mass%) is composed of carbon.

A BET specific surface area of the porous carbon material is not particularly limited, and surprisingly, it has been found that a smaller value of the BET specific surface area of the support of the present aspect is more preferable. Specifically, the BET specific surface area of the support is preferably 3000 m²/g or less, more preferably 2000 m²/g or less, still more preferably 1000 m²/g or less, and particularly preferably 500 m²/g or less. On the other hand, a lower limit value of the BET specific surface area is not particularly limited, and is usually 10 m²/g or more, preferably 50 m²/g or more, and more preferably 60 m²/g or more. That is, in a preferred embodiment of the present embodiment, the support is a porous carbon material having a BET specific surface area of 50 to 3000 m²/g. With such a configuration, excellent catalytic activity can be exhibited. The BET specific surface area of the porous carbon material can be determined by a BET method from a measurement result of the nitrogen adsorption/desorption isotherm.

Specific examples of the porous carbon material include graphene meso-sponge (GMS), carbon meso-sponge (CMS), activated carbon (YP-50F), zeolite templated carbon (ZTC), Ketjen Black, Vulcan, Denka Black (DB), and the like. Among them, as the porous carbon material, Denka black, Vulcan, Ketjen Black, or activated carbon is preferable, and Denka black or Vulcan is more preferable. Note that conventionally known knowledge can be appropriately referred to for a method for producing CMS or GMS.

The method for producing the catalyst support according to the present aspect is not particularly limited, and those skilled in the art can appropriately produce the catalyst support by considering technical common knowledge in the present technical field in addition to the section of Examples described later.

### <<Catalyst for Gas Phase Oxidation of Organic Compound>>

The above-described catalyst support according to one aspect of the present invention is used, for example, for gas phase oxidation of an organic compound. That is, another aspect of the present invention is a catalyst for gas phase oxidation of an organic compound including the above-described catalyst support according to one aspect of the present invention. Conventionally, it has been proposed to oxidize alcohol in a liquid phase system using the above-described compound as a catalyst. However, in a case of a reaction in the liquid phase system, it is necessary to separate a target product and a catalyst from a solvent after completion of a reaction and further purify the target product, which has been a problem in terms of time and cost. On the other hand, the catalyst for gas phase oxidation of an organic compound according to the present aspect enables oxidation of an organic compound in a gas phase system, and has an advantage that the above-described problem associated with performing a reaction in a liquid phase system does not occur. The organic compound which is a target of oxidation by the catalyst for gas phase oxidation according to the present aspect will be described later.

### <<Method for Gas Phase Oxidation of Organic Compound>>

According to still another aspect of the present invention, a method for gas phase oxidation of an organic compound is also provided. Specifically, the method is a method for gas phase oxidation of an organic compound, including: bringing an organic compound having an oxidizable functional group into contact with the catalyst support according to one aspect of the present invention described above in a gas phase in which molecular oxygen exists to oxidize the oxidizable functional group.

### <Organic Compound>

In the method for gas phase oxidation according to the present aspect, a target to be oxidized is an organic compound having an oxidizable functional group. The oxidizable functional group is not particularly limited, and examples thereof include a hydroxy group, a thiol group, and the like, and a hydroxy group is preferable. Further, since the oxidation method according to the present aspect is performed in a gas phase system, the organic compound may be a volatile organic compound (VOC). Examples of the organic compound include alcohols such as a primary alcohol and a secondary alcohol, mercaptans, and the like. By the method for gas phase oxidation according to the present aspect, the primary alcohol is oxidized to a corresponding aldehyde, the secondary alcohol is oxidized to a corresponding ketone, and the mercaptan is dimerized and oxidized to a corresponding disulfide. Among them, from the viewpoint that the reaction proceeds easily, the organic compound is preferably an alcohol, and more preferably a primary alcohol or a secondary alcohol.

### <Reaction Conditions>

In reaction conditions for carrying out the method for gas phase oxidation according to the present aspect, any other specific configurations are not particularly limited as long as the reaction conditions are in the gas phase in which molecular oxygen exists. The "gas phase in which molecular oxygen exists" can be realized, for example, by supplying molecular oxygen into the reaction system. Also, although pure oxygen gas may be used as a supply source of molecular oxygen, it is also possible to perform the reaction using an oxygen-containing mixed gas in which an oxygen content is diluted to about 5 to 50 vol% with an inert gas such as nitrogen gas or carbon dioxide gas, or air. The reaction system is not particularly limited, and may be a sealed system, a flow system, or an open system (in the atmosphere).

The reaction temperature can be constant or can be changed. In a case in which the reaction temperature is constant, the reaction temperature is not particularly limited, and for example, is preferably 0°C or higher, more preferably 10°C or higher, still more preferably 15°C or higher, and particularly preferably 20°C or higher. When the temperature is 0°C or higher, the reactivity of the oxidation reaction can be enhanced. On the other hand, the reaction temperature is preferably 100°C or lower, more preferably 80°C or lower, still more preferably 50°C or lower, and particularly preferably 40°C or lower. When the reaction temperature is 100°C or lower, deterioration of the catalyst support can be suppressed. In addition, in a case in which the reaction temperature is changed, the reaction temperature can be raised, lowered, or repeatedly raised and lowered.

A reactor for the gas phase oxidation reaction is not particularly limited as long as a target gas can be brought into contact with the catalyst support (catalyst for gas phase oxidation), and any one of a flow type reactor, a full batch type reactor, and a semi-batch type reactor can be used. Examples of the flow type reactor include a fixed-bed normal-pressure flow type reactor and a fluidized bed reactor. In a fixed-bed normal-pressure flow type reactor, a fixed layer in which a catalyst is formed into particles and fixed is used. In a fluidized bed reactor, a moving bed for moving a catalyst is used. From the viewpoint of efficiently bringing a reaction gas into contact with a catalyst, it is preferable to use a fixed-bed normal-pressure flow type reactor. In the reactor as described above, the catalyst may be externally heated by an electric heater, infrared rays, or the like.

The gas phase oxidation method of the organic compound using the catalyst for gas phase oxidation described above is not limited to the above-described embodiment, and for example, an embodiment in which the catalyst for gas phase oxidation is disposed in a wind direction louver installed in a blowout port of an air conditioner of an automobile (air conditioner), a filter installed in front of a blowout port, or the like may be adopted. According to such an embodiment, an organic compound contained in the air cooled or heated in an air conditioner and discharged from a blowout port can be oxidized. An organic compound (for example, alcohol) having a unique odor may be contained in the air discharged from an air conditioner, but according to the above embodiment, the odor can be reduced by oxidizing such an organic compound.

### <<Catalyst-Containing Base material and Spray>>

According to still another aspect of the present invention, there is also provided a catalyst-containing base material in which the catalyst support according to one aspect of the present invention described above is supported or coated on a base substrate. By using such a catalyst-containing base material, the catalyst support can be easily used for various applications.

Examples of the base substrate constituting the catalyst-containing base material include woven fabrics, nonwoven fabrics, filters, urethane foams, and the like as materials which are inexpensive and on which a catalyst support can be easily supported or coated. A method for supporting or coating these base substrates on the catalyst support is not particularly limited. Such a catalyst-containing base material can be suitably used for applications such as a mask, a medical textile product, an air conditioner, an air cleaner, a refrigerator, a temperature controller, a dehumidifier, a humidifier, or a filter for a suction vacuum cleaner. It goes without saying that the catalyst-containing base material may be used for other applications.

As yet another aspect of the present invention, a spray is also provided. The spray includes a spray container; and a solution which is contained in the spray container and in which the catalyst support is dispersed. The spray according to the present aspect can oxidize an organic compound at a desired site by being sprayed to the site, and can exhibit an effect of reducing odor of the organic compound, for example.

Note that the following embodiments are also included in the scope of the present invention: the catalyst support according to claim 1 having the feature of claim 2; the catalyst support according to claim 1 or 2 having the feature of claim 3; the catalyst support according to any one of claims 1 to 3 having the feature of claim 4; the catalyst support according to claim 3 or 4 having the feature of claim 5; the catalyst support according to any one of claims 1 to 5 having the feature of claim 6; the catalyst support according to any one of claims 1 to 6 having the feature of claim 7; the catalyst support according to any one of claims 1 to 7 having the feature of claim 8; the catalyst support according to any one of claims 1 to 8 having the feature of claim 9; the catalyst support according to any one of claims 1 to 9 having the features of claim 10; the catalyst support according to any one of claims 1 to 10 having the features of claim 11; the catalyst support according to any one of claims 1 to 11 having the features of claim 12; the catalyst for gas phase oxidation of an organic compound including the catalyst support according to any one of claims 1 to 12; a method for gas phase oxidation of an organic compound using the catalyst support according to any one of claims 1 to 12; the method according to claim 14 having the features of claim 15; the method according to claim 14 or 15 having the feature of claim 16; a catalyst-containing base material using the catalyst support according to any one of claims 1 to 12; the catalyst-containing base material according to claim 17 having the feature of claim 18; the catalyst-containing base material according to claim 17 or 18 having the feature of claim 19; and a spray using the catalyst support according to any one of claims 1 to 12.

### Examples

Hereinafter, embodiments of the present invention will be described in detail using examples, but the technical scope of the present invention is not limited to only the following examples.

### <<Preparation Example of Catalyst Support>>

### (Preparation of AZADO/GMS, AZADOL/GMS, and NHPI/GMS)

5 mg (0.13 mmol) of AZADO and 100 mL of dichloromethane were added to a 300 mL eggplant flask, AZADO was dissolved with ultrasonic waves, 100 mg of GMS (derived from SBa-200, S_{BET} = 1692 m²/g) as a support was added thereto, and dispersed with ultrasonic waves for 30 minutes, then stirred at room temperature for 30 minutes. Thereafter, the mixture was evaporated to dryness at 40°C and 600 hPa using a rotary evaporator and further dried under vacuum for 10 hours to obtain a catalyst support (AZADO/GMS). In addition, catalyst supports (AZADOL/GMS and NHPI/GMS) were prepared using, as organic molecular catalysts, 2-azaadamantane-N-hydroxyl (AZADOL) in which a nitroxyl radical of AZADO was changed to an alcohol and N-hydroxyphthalimide (NHPI) other than AZADO, respectively.

### (Preparation of AZADO-Metal salt/GMS)

0.13 mmol (CuI: 25.0 mg, Cu(NO₃)₂·3H₂O: 31.7 mg, or Fe(NO₃)₃·9H₂O: 53.1 mg) of a metal salt and 200 mL (each 50 vol%, 200 mL of acetonitrile in a case of using CuI as the metal salt) of water/ethanol were added in a 300 mL eggplant flask to dissolve the metal salt with ultrasonic waves, and 400 mg of GMS as a support was added thereto and dispersed with ultrasonic waves for 30 minutes, then the mixture was stirred at room temperature for 30 minutes. Thereafter, the mixture was evaporated to dryness at 60°C and 100 hPa (60°C, 250 hPa in a case of using acetonitrile as an impregnation solution) using a rotary evaporator, and dried under vacuum for 10 hours to obtain metal salt-supported GMS.

Subsequently, 5 mg (0.0328 mmol) of AZADO and 100 mL of dichloromethane were added to a 300 mL eggplant flask, AZADO was dissolved with ultrasonic waves, a metal salt-supported GMS prepared in advance was added so that a substance amount of each metal ion was equal to a substance amount of AZADO (CuI/GMS: 102.2 mg, Cu(NO₃)₂/GMS: 102.5 mg, and Fe(NO₃)₃/GMS: 101.9 mg), and the mixture was dispersed with ultrasonic waves for 30 minutes, and then stirred at room temperature for 30 minutes. Thereafter, the mixture was evaporated to dryness at 40°C and 600 hPa using a rotary evaporator and further dried under vacuum for 10 hours to obtain catalyst supports (AZADO-CuI/GMS, AZADO-Cu(NO₃)₂/GMS, and AZADO-Fe(NO₃)₃/GMS) on which the cocatalyst was supported together with AZADO.

### (Preparation of AZADO-Cu(bpy)/GMS)

0.03 mmol (10.9 mg) of Cu(OTf)₂, 0.03 mmol (4.7 mg) of bpy, and 0.03 mmol (4.6 mg) of AZADO were each added to a 5 mL vial, dissolved in 3 mL of acetonitrile, and added to a 300 mL eggplant flask containing 90 mL of acetonitrile. After each reagent was added, the vial wall was washed with acetonitrile and the result was added to the flask. Subsequently, 100 mg of GMS as a support was added to a flask and dispersed with ultrasonic waves for 30 minutes, then the mixture was stirred at room temperature for 30 minutes. Thereafter, the mixture was evaporated to dryness at 60°C and 250 hPa using a rotary evaporator and further dried under vacuum for 10 hours to obtain a catalyst support (AZADO-Cu(bpy)/GMS) on which a cocatalyst was supported together with AZADO. A coverage of the catalyst support thus obtained was 8%.

### (Preparation of AZADO-Cu(bpy) Catalyst Supports Using Various Carbon Materials and Oxides as Supports)

A catalyst support was prepared by the same procedure as in the above "Preparation of AZADO-Cu(bpy)/GMS". As a support, activated carbon (YP-50F, S_{BET} = 1700 m²/g), zeolite templated carbon (ZTC, S_{BET} = 3790 m²/g), carbon mesosponge (CMS, S_{BET} = 2150 m²/g), Ketjen Black (registered trademark) EC300J (KB, S_{BET} = 800 m²/g), Vulcan (registered trademark) XC-72 (VC, S_{BET} = 220 m²/g), and Denka Black (registered trademark) (DB, S_{BET} = 68 m²/g) were used as carbon materials in addition to GMS. Also, silica gel (Wakosil (registered trademark) C-300, S_{BET} = 475 m²/g), fumed silica (S_{BET} = 395 m²/g), aluminum oxide (TM-100, S_{BET} = 120 m²/g), titanium oxide (S_{BET} = 46 m²/g), magnesium oxide (S_{BET} = 60 m²/g), cerium oxide (S_{BET} = 70 m²/g), and zeolite (HSZ-320NAA, S_{BET} = 660 m²/g) were used as the oxides as a support. A coverage of the catalyst support thus obtained was 8% (YP-50F), 2% (ZTC), 6% (CMS), 16% (KB), 60% (VC), 193% (DB), 28% (silica gel), 33% (fumed silica), 109% (aluminum oxide), 285% (titanium oxide), 219% (magnesium oxide), 187% (cerium oxide), and 20% (zeolite).

### <<Evaluation of Physical Properties of Catalyst Support>>

### [Structural Analysis and Evaluation of Catalytic Activity of AZADO/GMS]

### (Structural Analysis of AZADO/GMS)

### · XRD Measurement

The AZADO/GMS prepared above was subjected to XRD measurement to analyze a structure of the catalyst. GMS and AZADO were also measured in the same manner. MiniFlex600 manufactured by Rigaku was used as a measuring apparatus, and a CuKα ray (1.5406 Å) was used as an X-ray source. The measurement was performed by uniformly spreading a sample in a circular recess having a diameter of 5 mm of a Si non-reflective sample plate. The measurement conditions are as follows:

| | |
|---|---|
| X-rays | Tube: Cu |
| | Tube voltage: 40 kV |
| | Tube current: 15 mA |
| Slit | DS: 1.25 deg. |
| | RS: 13.0 mm |
| | SS: 8.0 mm |
| Measurement | Detector: D/tex Ultra |
| | Sampling width: 0.02 deg. |
| | Scan speed: 10.0 deg./min |
| | Scanning axis: 2θ/θ |
| | Measurement range: 2 to 60 deg. |

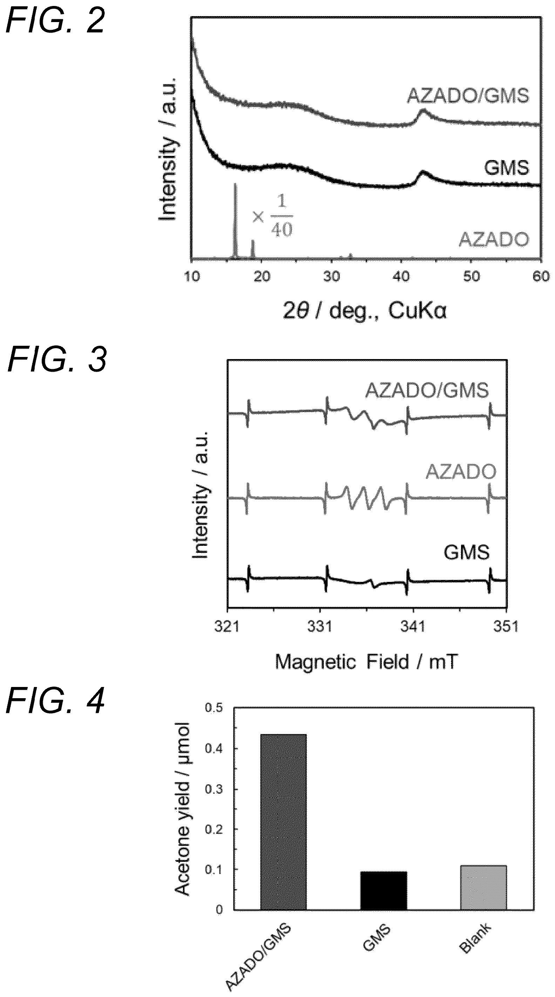

The obtained XRD pattern is shown in FIG. 2. In the XRD pattern of GMS, peaks of carbons (002) and (10) were observed near 20 = 26° and 44°, respectively. Also, in the XRD pattern of AZADO, sharp peaks were observed around 16.2° and 18.7°. In AZADO/GMS, an XRD pattern equivalent to that in GMS was obtained, indicating that AZADO was highly dispersively supported on GMS.

### · ESR measurement

ESR measurement was performed on the AZADO/GMS (2% in Al₂O₃) prepared above, and an existence state of AZADO on the GMS was analyzed. Measurement was similarly performed for 10 µM AZADO toluene solution and GMS (2% in Al₂O₃). JES-X330 manufactured by JEOL was used as a measuring apparatus. A powdered catalyst sample was diluted 50 times by mixing with an alumina powder using an agate mortar, and 100 mg of the diluted catalyst sample was filled in a quartz/Pyrex (registered trademark) sample tube having an inner diameter of 4 mm and a length of 180 mm, and the measurement was performed. AZADO was dissolved in toluene to obtain a 10 µM toluene solution, and 600 µL of the solution was filled in the same sample tube as in the case of the powder sample to perform the measurement. The measurement conditions are as follows:
Sweep magnetic field range: 336 ± 15 mT
Sweep time: 1.0 min
Microwave power: 1 mW
Time constant: 0.03 s
Modulated magnetic field frequency: 100 kHz
Modulated magnetic field: 0.2 mT
Amplification rate: 100
Insertion amount of Mn marker: 800

The obtained ESR spectrum is shown in FIG. 3. In the ESR spectrum of the AZADO toluene solution, three peaks derived from unpaired electrons of AZADO were observed. In addition, in the ESR spectrum of GMS diluted with alumina, one weak peak derived from an unpaired electron of GMS was observed. In the ESR spectrum of alumina-diluted AZADO/GMS, a peak considered to be superposition of a signal derived from AZADO and a signal derived from GMS was observed, suggesting that AZADO exists as a radical species on GMS.

### (Evaluation of Catalytic Activity by Gas Phase Oxidation Reaction of 2-Propanol in Batch System)

The gas phase oxidation reaction of 2-propanol was performed at 80°C in a batch system using AZADO/GMS prepared above as a catalyst. In addition, in a case in which only GMS was used as a catalyst and in a case in which no catalyst was used (Blank), the reaction was performed in the same manner. Here, 30 mg of AZADO/GMS and 20 mg of GMS were used. An acetone production amount at 24 hours after the start of a reaction is shown in FIG. 4. In a case in which AZADO/GMS was used as a catalyst, acetone production of 0.43 µmol (0.29 µmol in terms of 20 mg of the catalyst) was observed at 24 hours after the start of a reaction. The turnover number of the catalyst at this time (Turnover Number; TON) was calculated to be 0.05, indicating that the catalytic activity of AZADO/GMS is very low. In a case in which GMS was used as a catalyst and in a case in which no catalyst was used, the acetone production amounts at 24 hours after the start of a reaction were 0.094 µmol and 0.11 µmol, respectively, and it was found that the gas phase oxidation reaction of 2-propanol was not promoted only by GMS.

### [Structural Analysis and Evaluation of Catalytic Activity of AZADO-Cu(NO₃)₂/GMS]

### (Structural analysis of AZADO-Cu(NO₃)₂/GMS)

### · XRD Measurement

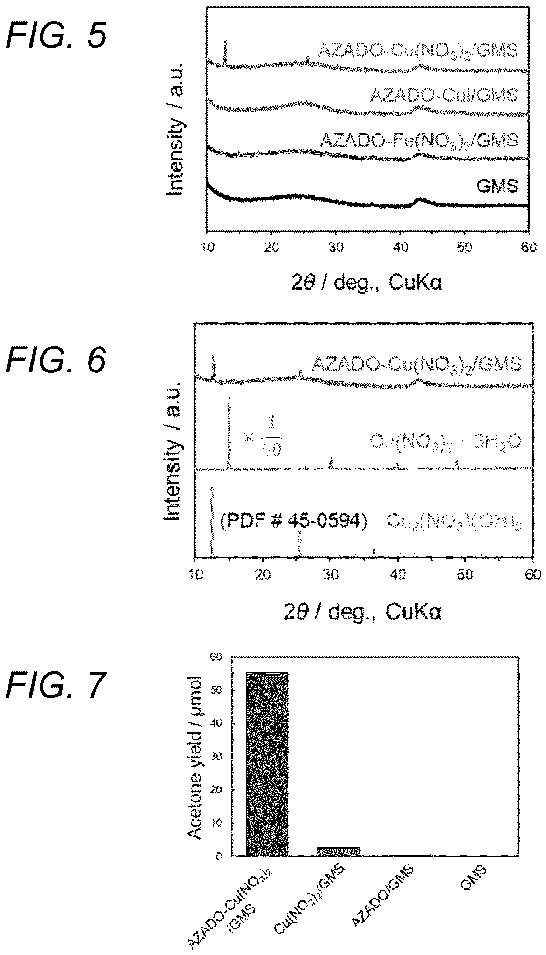

The AZADO-Cu(NO₃)₂/GMS, AZADO-CuI/GMS, and AZADO-Fe(NO₃)₃/GMS prepared above were subjected to XRD measurement to analyze the structure of the catalyst. The obtained XRD pattern is shown in FIG. 5. In AZADO-CuI/GMS and AZADO-Fe(NO₃)₃/GMS, patterns equivalent to those of GMS were obtained, and it was confirmed that the AZADO and the metal cocatalyst were highly dispersively supported on the GMS. In AZADO-Cu(NO₃)₂/GMS, peaks were observed at around 12.7° and 25.6°. However, these peaks did not show any correspondence with those seen in Cu(NO₃)₂·3H₂O (FIG. 6). Therefore, when the obtained XRD pattern was collated with the database, it was found that these two peaks were derived from Cu₂(NO₃)(OH)₃ (PDF#45-0594) (FIG. 6). From this, it was found that Cu₂(NO₃)(OH)₃ was formed on AZADO-Cu (NO₃)₂/GMS.

### · Elemental Analysis

CHN elemental analysis and ICP emission spectroscopic analysis were performed on AZADO-Cu(NO₃)₂/GMS prepared above to analyze the contents of carbon (C), hydrogen (H), nitrogen (N), and copper (Cu) in the catalyst. Here, it was assumed that GMS was composed only of carbon, and AZADO and Cu(NO₃)₂ added during the catalyst preparation were all supported on the GMS. As an analytical instrument for CHN element analysis, MICRO CORDER JM10 manufactured by J Science Lab was used. The analysis conditions are as follows:

| | |
|---|---|
| Bridge current | H: 91 mA |
| | C: 68 mA |
| | N: 125 mA |
| Furnace temperature | SF: 950°C |
| | CF: 830°C |
| | RF: 550°C |
| Flow rate | He: 180 mL/min |
| | O: 17 mL/min |
| Combustion time | : 240 seconds |

Also, an inductively coupled plasma (ICP) atomic emission spectrometer (SPS-3500 manufactured by Hitachi High-Tech Science Corporation) was used as an analytical instrument for ICP atomic emission spectrometry.

As a result of the analysis, it was found that about 67% of the added Cu was supported on the GMS from a theoretical value and a measured value of a content of each element calculated from the charged amount. Considering that the theoretical amount of N derived from AZADO is 0.4 wt%, and assuming that nitrate ions (NO³⁻) are supported as Cu(NO₃)₂ it was estimated that 100% of the added AZADO was supported on GMS. In addition, the measured value of a H content was larger than the theoretical value, and it was estimated that the formation of Cu₂(NO₃)(OH)₃ described above was the main factor.

### (Evaluation of Catalytic Activity)

### · Gas Phase Oxidation Reaction of 2-Propanol in Batch System

The gas phase oxidation reaction of 2-propanol was performed in a batch system using the AZADO-Cu (NO₃)₂/GMS, Cu(NO₃)₂/GMS, and AZADO/GMS prepared above as catalysts. Specifically, 20 mg of the catalyst support was weighed in a 100 mL reagent bottle, and the bottle was capped with a screw cap dedicated to a dehydrated solvent. Thereafter, O₂ including 1.8 mmol/L of 2-propanol was introduced at 50 mL/min for 15 minutes, and the reagent bottle was allowed to stand in an incubator kept at 30°C to carry out a reaction for 24 hours. In a case in which AZADO-Cu(bpy)/GMS was used as a catalyst, a similar reaction test was performed using N₂ or synthetic air instead of O₂. In a case AZADO/GMS was used as a catalyst, 20 mg of the catalyst was weighed in a 50 mL vial, the vial was covered with a septum, and a parafilm was wound, and then, O₂ including 2-propanol at 0.7 mmol/L was introduced at 50 mL/min for 15 minutes, and the lower part of the vial was immersed in an oil bath at 80°C to perform a reaction for 24 hours. In a case in which AZADO-Cu(NO₃)₂/GMS was used as a catalyst, 20 mg of the catalyst was weighed in a 100 mL reagent bottle, and the bottle was capped with a screw cap dedicated to a dehydrating solvent, then O₂ including 2-propanol at 0.7 mmol/L was introduced at 50 mL/min for 15 minutes, and the lower part of the reagent bottle was immersed in an oil bath at 80°C or 30°C to perform a reaction for 24 hours. In a case in which AZADO/GMS and AZADO-Cu(NO₃)₂/GMS were used as catalysts, the bubbler was not heated in an oil bath, and not the entire reaction vessel but only the lower part thereof was heated.

At the start of a reaction and after a certain period of time, 1 mL of a reaction gas was collected using a gas-tight syringe and analyzed by gas chromatography. A flame ionization detector (FID) was used as a detector. The reaction conditions and analysis conditions are as follows:

### (Reaction Conditions)

Catalyst support 80°C: AZADO/GMS 30 mg
: AZADO-Cu(NO₃)₂/GMS, Cu(NO₃)₂/GMS, AZADO/GMS, and GMS 20 mg each
30°C: AZADO/GMS, AZADO-Cu(NO₃)₂/GMS, AZADO-CuI/GMS, AZADO-Cu(bpy)/GMS, TEMPO-Cu(bpy)/GMS, AZADO-Cu(bpy)/YP-50F, AZADO-Cu(bpy)/ZTC, AZADO-Cu(bpy)/CMS, AZADO-Cu(bpy)/KB, AZADO-Cu(bpy)/VC, AZADO-Cu(bpy)/DB, AZADO-Cu(bpy)/Silica gel, AZADO-Cu(bpy)/Fumed silica, AZADO-Cu(bpy)/Al₂O₃, AZADO-Cu(bpy)/TiO₂, AZADO-Cu(bpy)/MgO, AZADO-Cu(bpy)/CeO₂, AZADO-Cu(bpy)/Zeolite 20 mg each

| | | |
|---|---|---|
| Oxidizing agent | O₂ | |
| Reaction substrate | 2-propanol | |
| Reaction temperature | Oil bath | 80°C or 30°C |
| | Incubator | 30°C |
| (Analysis Conditions) | | |

**[Table 1]**

| GC Parameter | | Retention time | |
|---|---|---|---|
| Column temperature | : 85°C | Acetone | : 2.5 min |
| Injector temperature | : 85°C | 2-Propanol | : 5.5 min |
| Detector temperature | : 85°C | | |
| Inlet pressure | : 120 kPa | | |
| Spirit Ratio | : 50.0 | | |
| Sample size | : 1.0 mL | | |

An acetone production amount at 24 hours after the start of a reaction at 80°C is shown in FIG. 7. In a case in which GMS and GMS supported with AZADO were used as catalysts, proceeding of the oxidation reaction was hardly confirmed. In the GMS in which only Cu(NO₃)₂ was supported, slight acetone production of 2.6 µmol was confirmed. On the other hand, in the case of AZADO-Cu(NO₃)₂/GMS in which AZADO and Cu(NO₃)₂ were supported on GMS, significant acetone production of 55 µmol was confirmed, and TON showed 9.0. From these results, it was found that the oxidation reaction was promoted in concert when both AZADO and the cocatalyst (here, Cu(NO₃)₂ containing Cu²⁺) were supported on the support.

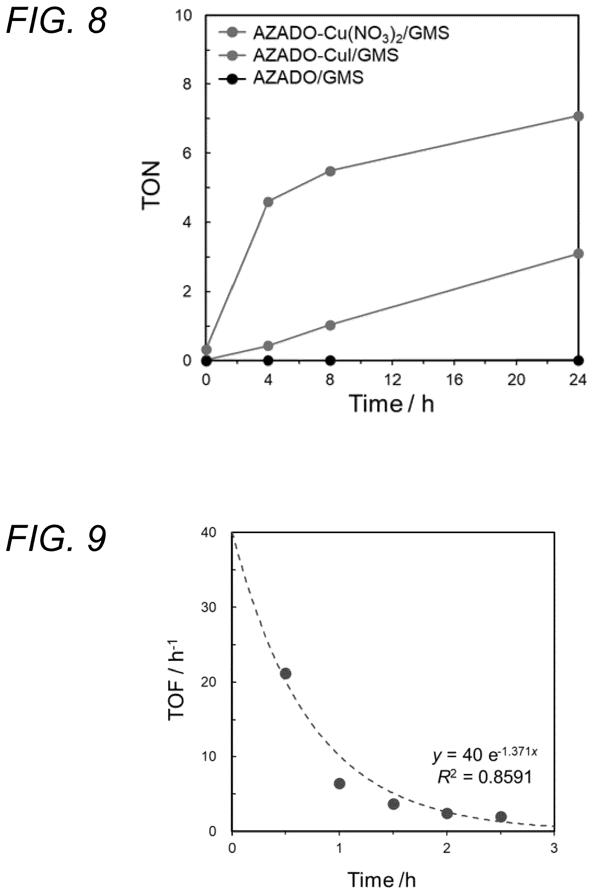

In the same manner as described above except that the reaction temperature was changed to 30°C, AZADO-CuI/GMS using CuI as a cocatalyst in addition to Cu(NO₃)₂ was applied to a gas phase oxidation reaction of 2-propanol at normal temperature (30°C) together with AZADO-Cu(NO₃)₂/GMS, and catalytic activities thereof were compared. A temporal change of the obtained TON is shown in FIG. 8. When AZADO-Cu(NO₃)₂/GMS was used, catalytic acetone production was confirmed even at 30°C. The reaction rate tended to decrease with the lapse of reaction time, and TON at 24 hours after the start of a reaction was 7.0. Also in AZADO-CuI/GMS, catalytic acetone production was observed, and the reaction rate showed a substantially constant value. TON at 24 hours after the start of a reaction was 3.1, and the activity was lower than that in a case of using Cu(NO₃)₂ as a cocatalyst.

It has been reported that in a reaction in a liquid phase system using AZADO as a catalyst, higher activity is obtained in a case in which copper (I) ions are used than in a case in which copper (II) ions are used. On the other hand, in the present reaction, the catalyst using Cu(NO₃)₂ containing divalent copper (II) ions exhibited higher activity than the catalyst using CuI containing monovalent copper (I) ions. From this, it was suggested that in the gas phase catalyst system, the reaction might proceed by a reaction mechanism different from that in the liquid phase system.

### · Gas Phase Oxidation Reaction of 2-Propanol in Flow System

In a reaction at 30°C in a batch system, when AZADO-Cu(NO₃)₂/GMS was used as a catalyst, a decrease in the reaction rate, which was considered to be caused by the feed rate control of 2-propanol, was observed. Therefore, an excessive amount of 2-propanol was supplied, and for the purpose of examining catalyst durability, a gas phase oxidation reaction of 2-propanol was performed at 24°C in a flow system. Specifically, O₂ including 1.8 mmol/L of 2-propanol was circulated at 50 mL/min in a glass tube having an outer diameter of 1/4 inch and containing 20 mg of AZADO-Cu(bpy)/DB, and the reaction was performed for 24 hours in an incubator in which the temperature was maintained at 30°C. In a case in which AZADO-Cu(NO₃)₂/GMS was used as a catalyst, 20 mg of the catalyst was used, and O₂ including 2-propanol at 1.0 mmol/L was circulated at 20 mL/min to perform a reaction at room temperature (24°C). In a case in which AZADO-Cu(bpy)/GMS was used as a catalyst, 20 mg of the catalyst was used, O₂ including 2-propanol at 1.3 mmol/L was circulated at 20 mL/min, and the lower part of the glass tube was immersed in an oil bath at 30°C to perform a reaction for 24 hours. At the start of a reaction and after a certain period of time, 1 mL of gas was collected from the glass tube downstream using a syringe and analyzed by gas chromatography. A flame ionization detector (FID) was used as a detector. The reaction conditions were as follows, and the analysis conditions were the same as described above:

### (Reaction Conditions)

| | |
|---|---|
| Catalyst support | AZADO-Cu (NO₃)₂/GMS 20 mg |
| | AZADO-Cu(bpy)/GMS 20 mg |
| | AZADO-Cu(bpy)/DB 10 mg |
| Oxidizing agent | AZADO-Cu(NO₃)₂/GMS and AZADO-Cu(bpy)/GMS O₂ Flow (20 mL/min) |
| | AZADO-Cu (bpy)/DB O₂ flow (50 mL/min) |
| Reaction substrate | 2-propanol |
| Reaction temperature | AZADO-Cu(NO₃)₂/GMS 24°C (room temperature) |
| | AZADO-Cu(bpy)/GMS 30°C (oil bath) |
| | AZADO-Cu(bpy)/DB 30°C (incubator) |

A temporal change of catalyst turnover frequency (Turnover Frequency; TOF) in a case of using AZADO-Cu(NO₃)₂/GMS as a catalyst is shown in FIG. 9. The TOF rapidly decreased after showing 21.2 at 30 minutes after the start of a reaction and decreased to 6.4 at 1 hour after the start of a reaction. Thereafter, the TOF gradually decreased to 2.0 at 2.5 hours after the start of a reaction. TON calculated based on FIG. 9 was 29.2, which was higher than TON (7.0) in the batch system, and an improvement in TON was confirmed.

### [Structural Analysis and Evaluation of Catalytic Activity of AZADO-Cu(bpy)/GMS]

### (Structural analysis of AZADO-Cu(bpy)/GMS)

### · XRD Measurement

The AZADO-Cu(bpy)/GMS prepared above was subjected to XRD measurement to analyze the structure of the catalyst. Cu(OTf)₂ and bpy were also measured in the same manner. As a result, the XRD pattern of AZADO-Cu(bpy)/GMS was equivalent to that of GMS, and it was confirmed that AZADO, bpy, and Cu(OTf)₂ were highly dispersively supported on GMS.

### · Elemental Analysis

CHN elemental analysis and ICP emission spectroscopy analysis were performed on the prepared AZADO-Cu(bpy)/GMS to analyze the contents of C, H, N, and Cu in the catalyst. Here, it was assumed that GMS was composed only of carbon, and AZADO, Cu(OTf)₂ and bpy added during catalyst preparation were all supported on GMS.

From a theoretical value and a measured value of the content of each element obtained from the charged amount, it was found that about 84% of the added Cu was supported on the GMS, and Cu could be supported in a larger amount than in the case of using Cu(NO₃)₂ as a cocatalyst (Cu supported amount: 67%). From the comparison of the N content, it was considered that the entire amount of AZADO was supported.

### (Evaluation of Catalytic Activity)

### · Gas Phase Oxidation Reaction of 2-Propanol in Batch System

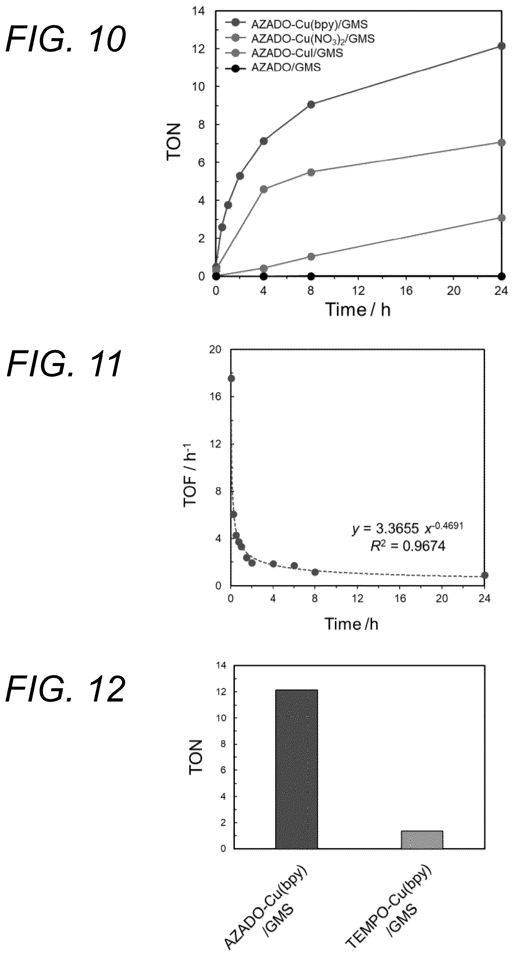

A temporal change of TON when the gas phase oxidation reaction of 2-propanol was performed at 30°C in a batch system using the AZADO-Cu(bpy)/GMS prepared above as a catalyst is shown in FIG. 10. In the figure, the reaction results of various catalysts (FIG. 8) are also shown for comparison. When AZADO-Cu(bpy)/GMS was used as a catalyst, the reaction proceeded steadily, and the reaction rate was higher than that in a case of using AZADO-Cu(NO₃)₂/GMS. Although the reaction rate tended to decrease, TON at 24 hours after the start of a reaction was 12.2, indicating higher catalytic activity than TON (7.0) in a case of using AZADO-Cu(NO₃)₂/GMS as a catalyst. As described above, in AZADO-Cu(NO₃)₂/GMS, Cu(NO₃)(OH)₃ is aggregated on GMS, whereas in AZADO-Cu(bpy)/GMS, AZADO and Cu(bpy) are highly dispersively supported on GMS. Therefore, it is presumed that in AZADO-Cu(bpy)/GMS, a ratio of the surface on which Cu is exposed increases, and the catalytic activity is improved.

### · Gas Phase Oxidation Reaction of 2-Propanol in Flow System

For the purpose of examining catalyst durability, the temporal change of TOF when a gas phase oxidation reaction of 2-propanol was performed at 30°C in a flow system using AZADO-Cu(bpy)/GMS as a catalyst is shown in FIG. 11. The TOF rapidly decreased after showing 17.6 at 3 minutes after the start of a reaction and decreased to 2.0 at 2 hours after the start of a reaction. However, the subsequent decrease in TOF was moderate, and TOF of 0.9 was shown at 24 hours after the start of a reaction. TON calculated on the basis of FIG. 11 was 34.3, which was higher than TON (12.2) in the batch system, and an improvement in TON was confirmed. Here, in a case in which the TOF at each reaction time in FIG. 11 was exponentially approximated, the value of the coefficient of determination (R²) decreased to 0.4491, so that TON was calculated using a function obtained by exponentially approximating the TOF.

### · Comparison between Catalytic Activities of AZADO and TEMPO

It is desirable that the catalyst for gas phase oxidation can also promote an oxidation reaction of a sterically intricate substrate. Therefore, TEMPO-Cu(bpy)/GMS using TEMPO as a nitroxyl radical was prepared by the same procedure as in the above "Preparation of AZADO-Cu(bpy)/GMS". Then, by applying to the gas phase oxidation reaction of 2-propanol and the gas phase oxidation reaction of ethanol together with AZADO-Cu(bpy)/GMS, the activity of each catalyst in both reactions was compared with each other.

First, TON's at 24 hours after the start of a reaction when a gas phase oxidation reaction of 2-propanol was performed at 30°C in a batch system using AZADO-Cu(bpy)/GMS and TEMPO-Cu(bpy)/GMS as catalysts are shown in FIG. 12. TEMPO-Cu(bpy)/AZADO showed a TON of 1.4 at 24 hours after the start of a reaction. On the other hand, AZADO-Cu(bpy)/GMS showed a TON of 12.2, representing approximately 9 times the catalytic activity of the catalyst with TEMPO as the nitroxyl radical. This showed that AZADO-Cu(bpy)/GMS was advantageous for oxidation of sterically intricate alcohols. However, it has been known that a secondary alcohol cannot be oxidized in a case in which TEMPO is used as a catalyst in a conventional liquid phase system. On the other hand, it was confirmed that the gas phase oxidation according to the present invention can oxidize the secondary alcohol even in a case in which TEMPO is used as a catalyst.

Subsequently, a gas phase oxidation reaction of ethanol was performed at 30°C in a batch system using AZADO-Cu(bpy)/GMS and TEMPO-Cu(bpy)/GMS as catalysts. Specifically, 20 mg of the catalyst was weighed in a 100 mL reagent bottle, and the bottle was capped with a screw cap dedicated to a dehydrated solvent. Thereafter, oxygen including ethanol at 2.5 mmol/L was introduced at 50 mL/min for 15 minutes, and the reagent bottle was allowed to stand in an incubator maintained at 30°C to carry out a reaction for 24 hours. At the start of a reaction and after a certain period of time, 1 mL of a reaction gas was collected using a gas-tight syringe and analyzed by gas chromatography. The reaction conditions and analysis conditions are shown below.

### (Reaction Conditions)

| | |
|---|---|
| Catalyst | AZADO-Cu(bpy)/GMS and TEMPO-Cu(bpy)/GMS 20 mg each |
| Oxidizing agent | O₂ |
| Reaction substrate | ethanol |
| Reaction temperature | 30°C (incubator) |
| (Analysis Conditions) | |

**[Table 2]**

| GC Parameter | | Retention time | |
|---|---|---|---|
| Column temperature | : 85°C | Acetaldehyde | : 1.5 min |
| Injector temperature | : 85°C | 2-Propanol | : 5.5 min |
| Detector temperature | : 85°C | | |
| Inlet pressure | : 120 kPa | | |
| Spirit Ratio | : 50.0 | | |
| Sample size | : 1.0 mL | | |

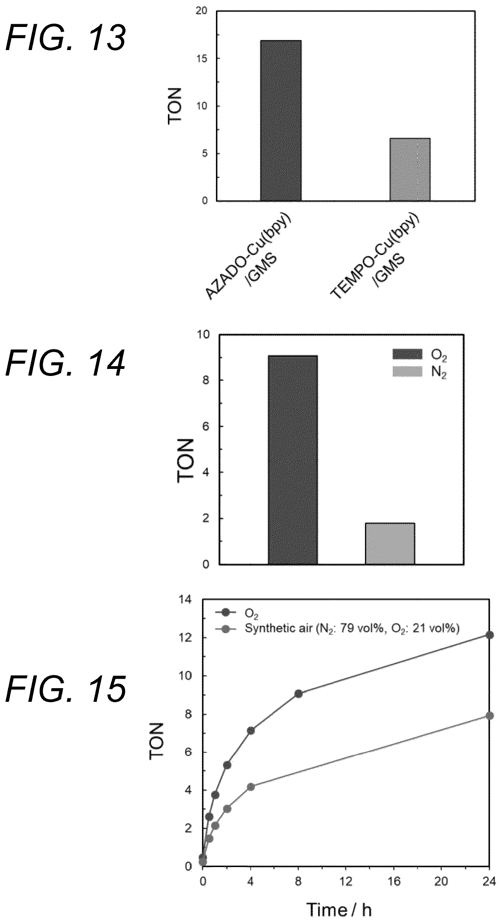

TON's at 24 hours after the start of a reaction are shown in FIG. 13. At 24 hours after the start of a reaction, AZADO-Cu(bpy)/GMS indicated TON of 17, and TEMPO-Cu(bpy)/GMS indicated TON of 6.6, and it was found that the catalyst using AZADO showed higher activity even in a case of using a sterically uncomplicated alcohol as a substrate.

### · Control Test

For the purpose of confirming that the oxidizing agent in the oxidation reaction of 2-propanol described above is oxygen, when N₂ including 1.8 mmol/L of 2-propanol was introduced into a reaction vessel, and a reaction was performed at 30°C for 8 hours in a batch system using AZADO-Cu(bpy)/GMS as a catalyst, TON at 8 hours after the start of a reaction is shown in FIG. 14. At 8 hours after the start of a reaction, the TON was 9.1 in the O₂ atmosphere, whereas the TON was 1.8 in the N₂ atmosphere, and the TON was reduced to about 1/5 as compared with the reaction in the O₂ atmosphere. From this, it was confirmed that O₂ acted as an oxidizing agent in the oxidation reaction of 2-propanol. Here, as a factor that TON did not become 0 even under the N₂ atmosphere, it is considered that replacement of air inside the reaction vessel with N₂ was insufficient, air was mixed at the time of introduction of N₂, air was mixed due to a decrease in internal pressure of the reaction vessel due to collection of a gas sample, and the like. In addition, it is considered that an oxidation reaction occurs by AZADO or AZADO⁺ even in the absence of O₂, and TON shows a maximum of 1. In this test, it is presumed that TON did not become 0 even under an N₂ atmosphere due to both of these contributions.

A temporal change of TON when introducing a synthetic air including 1.8 mmol/L of 2-propanol into a reaction vessel and performing a reaction at 30°C for 24 hours in a batch system using AZADO-Cu(bpy)/GMS as a catalyst is shown in FIG. 15. As a result, although the reaction rate was slower than that in a case of using O₂, the reaction proceeded steadily also in a case of using synthetic air. TON at 24 hours after the start of a reaction was 7.9, which was about 65% of that in a case of using O₂ (TON of 12.2). Although the ratio of O₂ contained in the synthetic air was about 1/5 of pure O₂, the activity of 65% in a case in which pure O₂ was introduced was obtained, and it was confirmed that the oxidation reaction was sufficiently promoted even under air.

### [Structural Analysis and Evaluation of Catalytic Activity of AZADO-Cu(bpy)-Based Catalysts Using Various Carbon Materials and Oxides as Supports]

### (Structural Analysis of AZADO-Cu(bpy)-Based Catalyst by XRD Measurement)

For catalysts prepared by supporting AZADO and Cu(bpy) on various carbon materials and oxides, XRD measurement was performed to analyze the structures of the catalysts. As a result, a pattern equivalent to that of the carbon material used was obtained in the XRD patterns of all catalyst, and it was confirmed that AZADO and Cu(bpy) were highly dispersively supported on each support.

In addition, for the catalysts prepared using various oxide supports, in the XRD patterns of all catalysts, the same pattern as that of the used oxide support was obtained, and it was confirmed that AZADO and Cu(bpy) were highly dispersively supported on each support.

### (Evaluation of Catalytic Activity)

### · Gas Phase Oxidation Reaction of 2-Propanol in Batch System

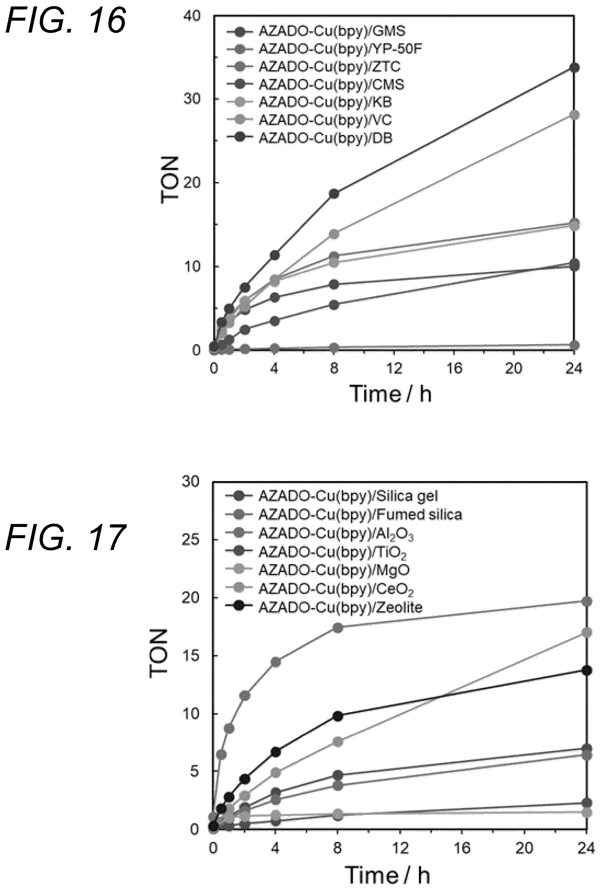

Temporal changes of TON obtained for a catalyst using various carbon materials as supports when performing a gas phase oxidation reaction of 2-propanol in a batch system using a catalyst in which AZADO and Cu(bpy) are supported on various carbon materials and oxides were shown in FIG. 16. TON in a case of using AZADO-Cu(bpy)/GMS, AZADO-Cu(bpy)/YP-50F, AZADO-Cu(bpy)/KB, or AZADO-Cu(bpy)/VC as a catalyst showed almost the same behavior up to 2 hours after the start of a reaction, and decreased with the lapse of the reaction time. Thereafter, in the catalyst using YP-50F or KB as a support, the reaction rate decreased with almost the same magnitude, and TON at 24 hours after the start of a reaction was 16 and 15, respectively. In the catalyst using GMS as a support, the decrease in reaction rate was larger than that in the catalysts using YP-50F and KB as supports, and TON at 24 hours after the start of a reaction showed 12. On the other hand, in the catalyst using VC as a support, a decrease in the reaction rate was hardly observed, and TON at 24 hours after the start of a reaction showed a high value of 28. In AZADO-Cu(bpy)/DB, the initial rate of the reaction was the highest, and a decrease in the reaction rate was hardly observed as in the case of using VC as a support. TON at 24 hours after the start of a reaction showed the highest value of 34. In AZADO-Cu(bpy)/CMS, the reaction rate until 4 hours after the start of a reaction was 1/2 or less of that of the catalyst using GMS, YP-50F, KB, VC, and DB as supports, but the reaction rate after 4 hours was not reduced and was almost constant, and TON at 24 hours after the start of a reaction was 10. Although the reaction proceeded in AZADO-Cu(bpy)/ZTC, TON at 24 hours after the start of a reaction showed a low value of 0.6.

Subsequently, the temporal change of TON obtained for catalysts using various oxides as supports is shown in FIG. 17. In AZADO-Cu(bpy)/MgO, the reaction proceeded, but the reaction rate was slow, and TON at 24 hours after the start of a reaction was 1. In AZADO-Cu(bpy)/TiO₂, the initial rate of the reaction was the lowest, but the reaction continued to proceed very slowly, and TON at 24 hours after the start of a reaction showed 2. In the catalysts using AZADO-Cu(bpy)/silica gel and AZADO-Cu(bpy)/fumed silica as supports, the reaction continued to proceed slowly, and TON at 24 hours after the start of a reaction was 7 and 6, respectively. In AZADO-Cu(bpy)/Al₂O₃, the reaction rapidly proceeded immediately after the start of a reaction. Although the reaction rate decreased exponentially thereafter, TON at 24 hours after the start of a reaction showed the highest value of 20. TON in a case of using AZADO-Cu(bpy)/zeolite also showed the same tendency as the catalyst using Al₂O₃ as a support, and TON at 24 hours after the start of a reaction showed a relatively high value of 14. In AZADO-Cu(bpy)/CeO₂, the initial rate of the reaction was lower than that in a case of using zeolite as a support, and a decrease in the reaction rate was hardly observed, and TON at 24 hours after the start of a reaction showed 17, which was higher than that in the case of using zeolite as a support, and was the second highest value in the case of using Al₂O₃ as a support. As a factor that the catalyst using Al₂O₃ as a support exhibits high catalytic activity, promotion of oxidation reaction by generation of oxoammonium ions is considered. As described above, the nitroxyl radical is oxidized to produce oxoammonium ions. In addition, Al₂O₃ is known to have solid acidity. It was inferred that disproportionation reaction of AZADO occurred due to solid acidity of Al₂O₃, and the generated oxoammonium ions promoted oxidation reaction. In a case of using CeO₂ as a support, the oxidation reaction may be promoted by the redox reaction of Ce.

### · Study of Support Effect

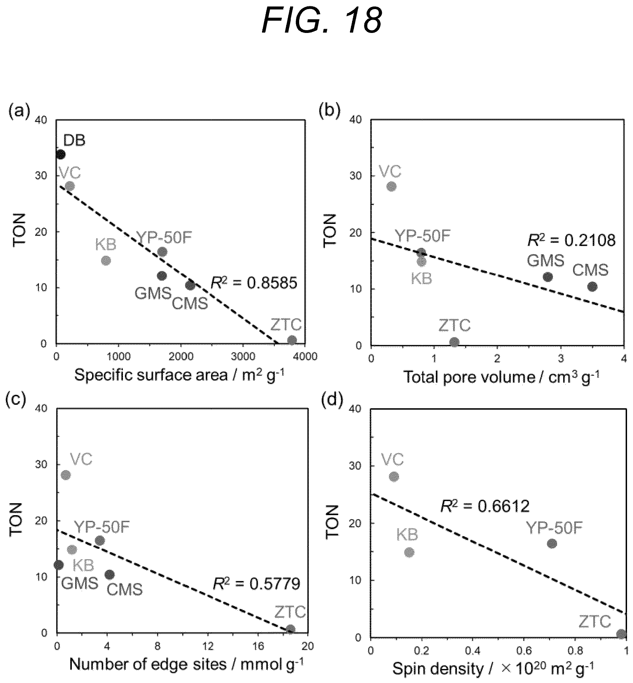

In the above study, it was shown that a gas phase oxidation reaction of 2-propanol was performed using a catalyst in which AZADO and Cu(bpy) were supported on various carbon materials, and the catalytic activity was changed depending on the carbon material used as a support. Therefore, TON against each of a specific surface area, a total pore volume, the number of edge sites, and a spin density of each carbon material at 24 hours after the start of a reaction obtained using catalysts using various carbon materials as supports was plotted, and correlation with TON was studied. The resulting plot is shown in FIG. 18. When plotting TON against the specific surface area, the obtained R² value was 0.8585, indicating a strong negative correlation therebetween (FIG. 18(a)). From this, it was presumed that AZADO and Cu(bpy) can exist close to each other on the carbon support having a low specific surface area, the oxidation reaction is efficiently promoted, and large TON is obtained. This is contrary to the conventional common sense that, in general, the larger the specific surface area of the catalyst support is, the more the catalyst support contributes to high catalytic activity. When TON was plotted against the total pore volume, the R² value was 0.2108, and it was found that there was almost no correlation between these values (FIG. 18(b)). In a case in which TON was plotted against the edge amount, the R² value was 0.5779, and it was found that a weak negative correlation was observed, but the correlation was small as compared with the correlation between the specific surface area and TON (FIG. 18(c)). In addition, in a case in which TON was plotted against spin density, the R² value was 0.6612, suggesting that there is a relatively strong negative correlation (FIG. 18(d)).

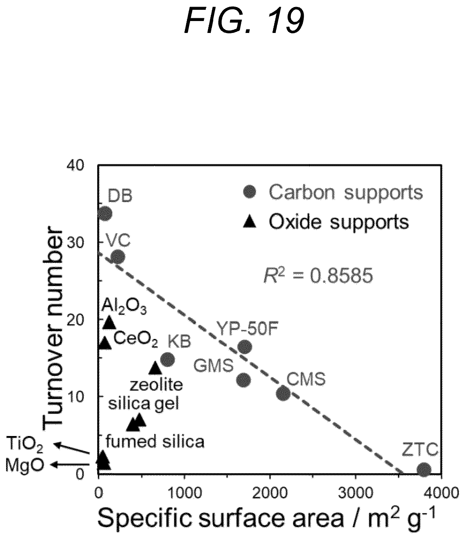

From the above studies, it was shown that there is a strong negative correlation between the activity of the catalyst using the carbon material as a support and the specific surface area of each carbon material. Therefore, TON at 24 hours after the start of a reaction obtained using catalysts using various oxides as supports was arranged by the specific surface area of each oxide, and compared with the case of using a carbon support. A plot of TON at 24 hours after the start of a reaction against the specific surface area of each oxide support is shown in FIG. 19. Here, FIG. 19 also shows plots in cases of using various carbon supports. From the obtained plots, it was found that even in a case of using any of oxide supports, the activity was lower than in a case of using the carbon support having an equivalent specific surface area. From this result, it was shown that in the present catalyst system using a solid catalyst supporting AZADO and Cu(bpy), a support composed of a carbon material was particularly useful. Among them, in a case in which Denka Black was used as a support, TON was 36, and the highest catalytic activity was obtained. This is presumed to be because the specific surface area of Denka Black was as small as 68 m²/g, and AZADO and Cu(bpy) could exist close to each other.

From the results shown above, it was shown that, according to the present invention, an organic compound can be oxidized in a gas phase using a nitroxyl radical as a catalyst.

The present application is based on Japanese Patent Application No. 2022-136100 filed on August 29, 2022, the disclosure content of which is incorporated herein by reference in entirety thereof.

## Claims

1. A catalyst support in which a compound having an oxidation-reduction mechanism below and a cocatalyst containing a transition metal are supported on a support, 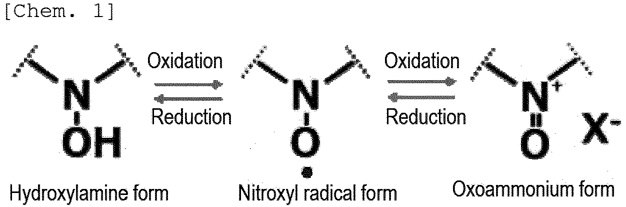 in the formula, X⁻ is a counteranion, and a broken line represents a bonding position with another atom.

2. The catalyst support according to claim 1, wherein a standard redox potential (25°C) between the nitroxyl radical form and the oxoammonium form is +100 mV to +1000 mV [Ag/Ag⁺].

3. The catalyst support according to claim 1, wherein the nitroxyl radical form of the compound is represented by Chemical Formula 1 below, 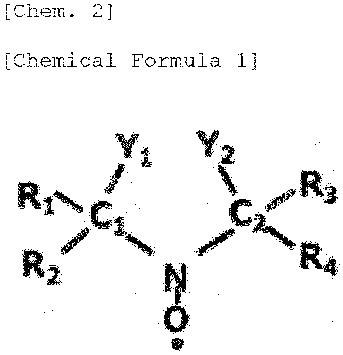 in the formula, R₁ to R₄, Y₁, and Y₂ are each independently a hydrogen atom or an optionally substituted monovalent organic group, in this case, Y₁ and Y₂ may be bonded to each other to form an optionally substituted nitrogen-containing monocyclic ring containing a 5- or 6-membered ring, and R₁ and R₃ may be bonded to each other to form an optionally substituted nitrogen-containing bicyclic ring containing a 5- or 6-membered ring, or two rings constituting the nitrogen-containing bicyclic ring may be further crosslinked to form an optionally substituted nitrogen-containing tricyclic ring containing a 5- or 6-membered ring.

4. The catalyst support according to claim 3, wherein the nitroxyl radical form of the compound is represented by Chemical Formula 2 below, 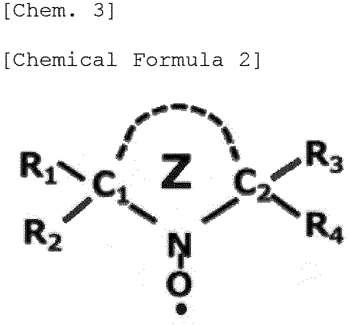 in the formula, R₁ to R₄ are each independently a hydrogen atom or an optionally substituted monovalent organic group and Z is an optionally substituted nitrogen-containing monocyclic ring containing a 5- or 6-membered ring, in this case, R₁ and R₃ may be further bonded to each other to form an optionally substituted nitrogen-containing bicyclic ring containing a 5- or 6-membered ring, or two rings constituting the nitrogen-containing bicyclic ring may be further crosslinked to form an optionally substituted nitrogen-containing tricyclic ring containing a 5- or 6-membered ring.

5. The catalyst support according to claim 3 or 4,
wherein in a case in which the compound does not contain the monocyclic ring, the bicyclic ring, or the tricyclic ring, or contains the nitrogen-containing monocyclic ring, R₁ to R₄ are each independently a hydrogen atom or an alkyl group having 1 to 4 carbon atoms, and
in a case in which the compound contains the nitrogen-containing bicyclic ring or the nitrogen-containing tricyclic ring, R₂ and R₄ are each independently a hydrogen atom or an alkyl group having 1 to 4 carbon atoms.

6. The catalyst support according to claim 1 or 2, wherein the nitroxyl radical form of the compound is one kind or two or more kinds selected from the group below consisting of:

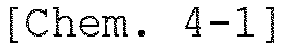

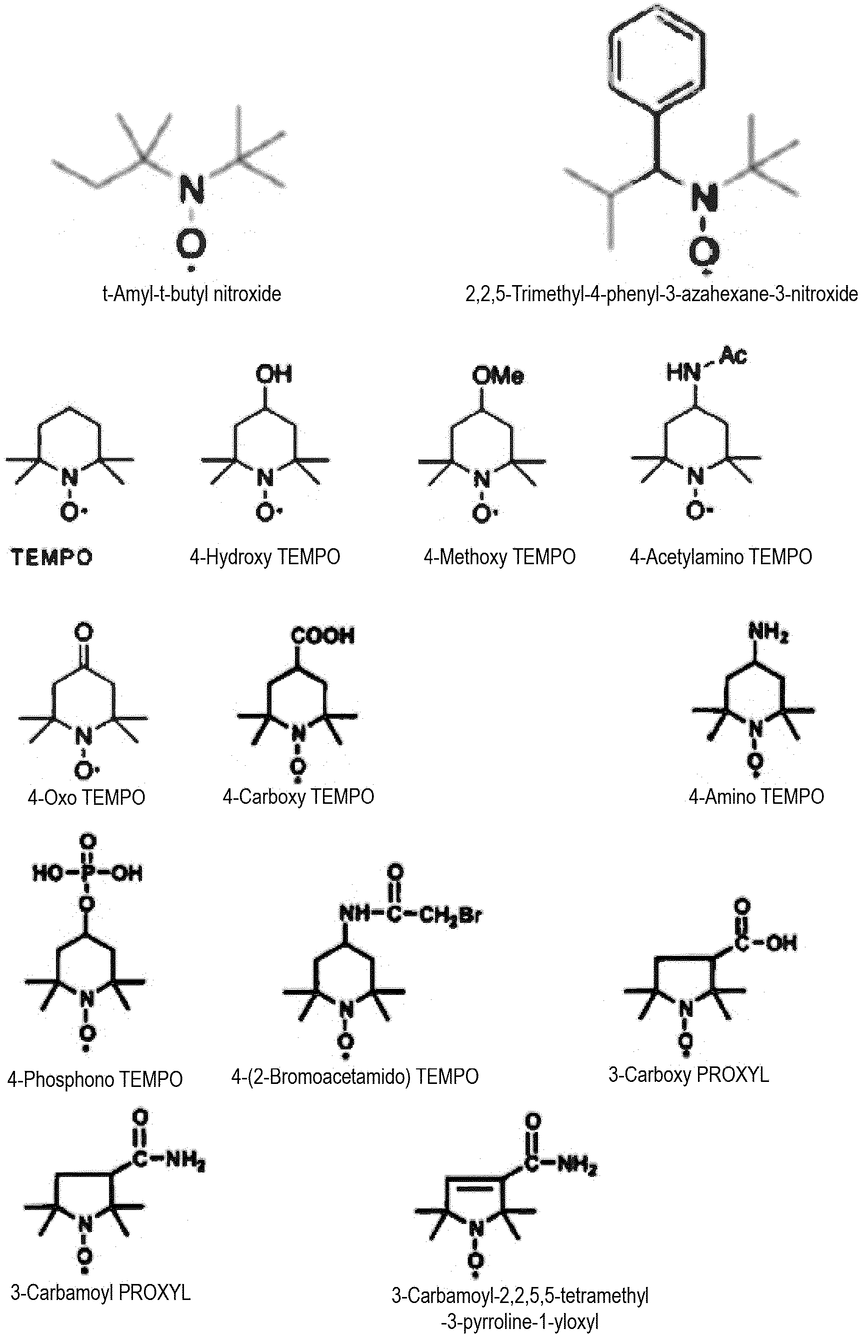

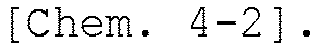

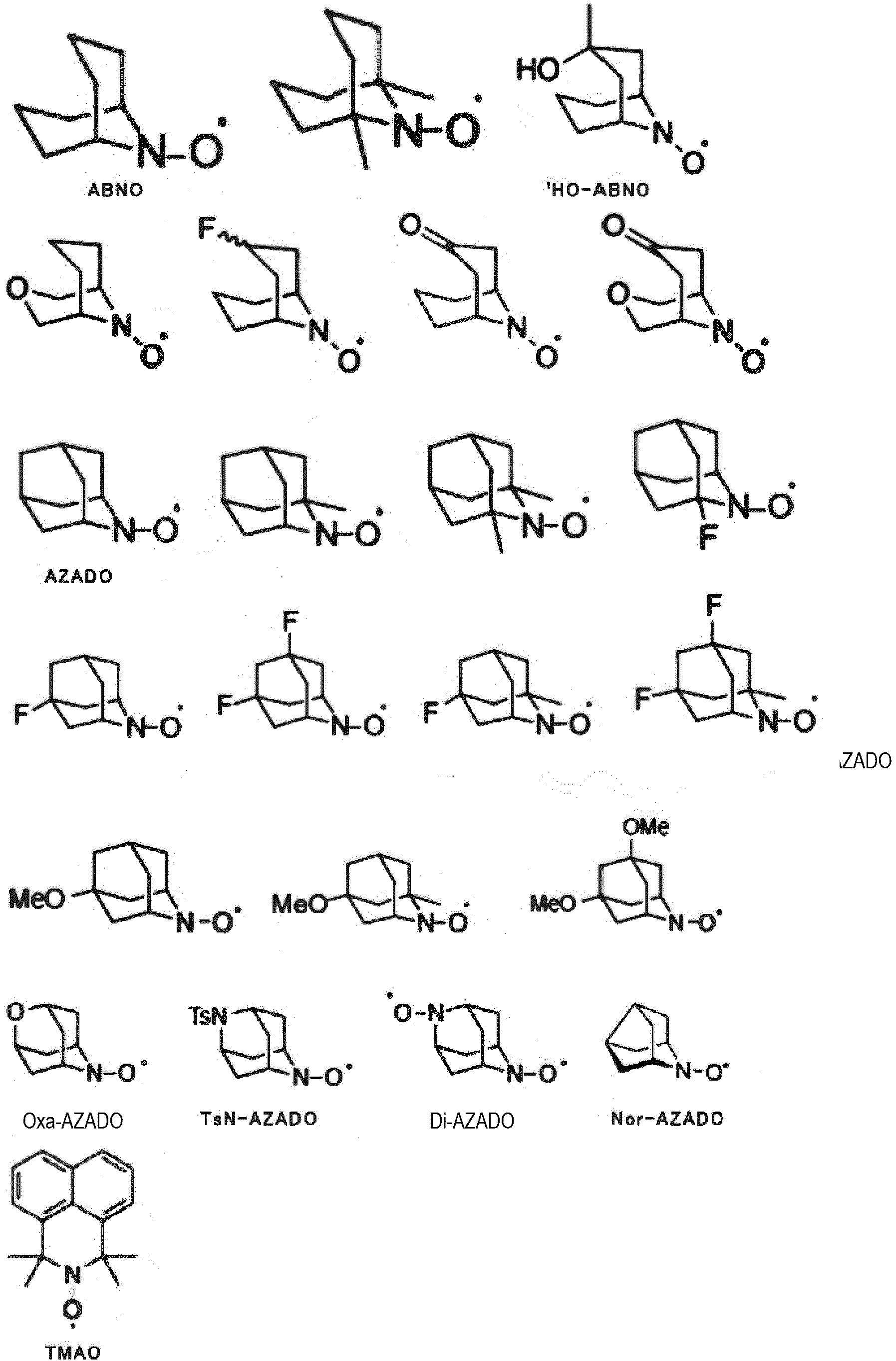

7. The catalyst support according to claim 6, wherein the nitroxyl radical form of the compound is 2-azaadamantane-N-oxyl (AZADO).

8. The catalyst support according to claim 1 or 2, wherein the transition metal includes one kind or two or more kinds selected from the group consisting of Ag, Au, Pt, Pd, Ni, Mn, Fe, Ti, Al, Zn, and Cu.

9. The catalyst support according to claim 8, wherein the transition metal includes Cu.

10. The catalyst support according to claim 1 or 2, wherein a molar ratio between supported amounts of the compound and the transition metal is 1:2 to 4:1 (compound:transition metal).

11. The catalyst support according to claim 1 or 2, wherein a coverage of a surface area of the support by the compound and the cocatalyst is 10% to 300%.

12. The catalyst support according to claim 1 or 2, wherein the support is a porous carbon material having a BET specific surface area of 50 to 3000 m²/g.

13. A catalyst for gas phase oxidation of an organic compound, comprising the catalyst support according to claim 1 or 2.

14. A method for gas phase oxidation of an organic compound, comprising: bringing an organic compound having an oxidizable functional group into contact with the catalyst support according to claim 1 or 2 in a gas phase in which molecular oxygen exists to oxidize the oxidizable functional group.

15. The method for gas phase oxidation according to claim 14, wherein the organic compound is a volatile organic compound.

16. The method for gas phase oxidation according to claim 14, wherein the organic compound is a primary alcohol or a secondary alcohol.

17. A catalyst-containing base material comprising the catalyst support according to claim 1 or 2 which is supported or coated on a base substrate.

18. The catalyst-containing base material according to claim 17, wherein the base substrate is a woven fabric, a non-woven fabric, a filter, or a urethane foam.

19. The catalyst-containing base material according to claim 17, which is used for a mask, a medical textile product, an air conditioner, an air cleaner, a refrigerator, a temperature controller, a dehumidifier, a humidifier, or a filter for a suction vacuum cleaner.

20. A spray comprising: a spray container; and a solution which is contained in the spray container and in which the catalyst support according to claim 1 or 2 is dispersed.
